# EUROPEAN PATENT APPLICATION

(11) **EP 4 119 032 A1**
(43) Date of publication of application: **18.01.2023**
(21) Application number: 22177646.1
(22) Date of filing: 05.10.2018
(51) Int. Cl.: A61B 1/018, A61B 1/00, A61B 1/015

(54) **AN ENDOSCOPE SYSTEM**

(30) Priority: 06.10.2017 SG 10201708265T
(62) Divisional of application: 18864205.2
(71) Applicant: EndoMaster Pte. Ltd., Singapore 119844 (SG); HOYA CORPORATION, Shinjuku-ku Tokyo 160-8347 (JP)
(72) Inventor: YAMAMOTO, Tomonori, 119844 Singapore (SG); NG, Boon Hang, 119844 Singapore (SG); PENNY, Isaac David, 119844 Singapore (SG); NAITO, Naoyuki, Tokyo, 160-8347 (JP); KOBAYASHI, Takahiro, Tokyo, 160-8347 (JP); HIRAYAMA, Tetsu, Tokyo, 160-8347 (JP)
(74) Representative: Grey, Ian Michael

(57) **Abstract**

An insufflation control device for an endoscope system is disclosed. It comprises a master valve that is disposed downstream relative to an insufflator of the endoscope system and upstream relative to an endoscope of the endoscope system. It further comprises a slave valve that is disposed downstream relative to the insufflator and upstream relative to the endoscope; and a valve controller that is configured to operationally control one or more of the master valve or the slave valve in response to commands received either from the endoscope system or from a remote console. The master valve and the slave valve are disposed in series with each other.

## Description

### FIELD OF INVENTION

The present invention relates broadly, but not exclusively, to an endoscope system.

### BACKGROUND

An endoscope is a hollow tube which is used to examine and/or deliver an instrument to an interior of a hollow organ or cavity of a body. For example, an endoscope can be used to examine the upper gastrointestinal tract (e.g., throat, esophagus or stomach) or the lower gastrointestinal tract (e.g. colon). The endoscope typically provides light to the internal area and provides vision for the endoscopist to navigate within the organ or cavity. Once an area is identified which needs treatment, an instrument necessary for treating the identified location is inserted into the hollow tube within the endoscope and maneuvered to the area. The instrument may, for example, be used to remove a polyp in the colon or to take a biopsy tissue sample form within the identified area for testing.

The camera which provides vision within the organ or cavity is on the endoscope and is oriented in the manner that the endoscope is oriented. The instrument is passed through the endoscope. As the endoscope may have multiple curves through which the instrument needs to pass, the insertion process may put rotational strain on the instrument. While the endoscope operator (or endoscopist) may have control over the end of the instrument outside the body, the rotational strain can release at any moment causing the distal end of the instrument at the identified location to rotate one way or another without control by the operator. As the location and the instrument orientation is critical for correct operation, the lack of control of rotation at the distal end is problematic. When the endoscope and instrument are operating under computer control, knowledge of the orientation of the instrument is system critical.

Conventional flexible endoscopes have removable mechanical valves that are attached on an endoscope control body. They also serve as input buttons for air feeding for insufflation, water feeding for lens cleaning, and vacuum pressure for suction. During endoscopy, a gastroenterologist keeps holding an endoscope and controls those input buttons by left hand as necessary. For a teleoperated endoscopic robot-assisted surgical system, it is preferable that those functions can be activated on the endoscope side as well as on the remote console side where the clinician (or endoscopist) teleoperates surgical instruments through joystick-like devices. To achieve this goal, one typical method involves using the mechanical valves on the conventional endoscope to be controlled directly and remotely. Alternatively, those valves can be taken out from the endoscope and replaced with electromechanical buttons, and another set of electromechanical buttons are implemented on the remote console side. Therefore, instead of using the mechanical valves, electromechanically-operated solenoid valves are housed in a separate box, called a valve control unit, outside the endoscope so that the valves can be controlled from both sides (as described in WO2016/148642).

In endoscopy, good insufflation is a key to obtain clear vision of a surgical site. Over-inflation, on the other hand, may cause serious injuries to the patients including perforation and embolism. The endoscopists typically rely on visual feedback from the endoscope camera to assess if the amount of insufflation is good or if they over-feed gas into the patient gastrointestinal (GI) tract because the currently available endoscopes do not have any indicators to show present air flow or total amount of gas pumped into the patient. Furthermore, experienced endoscopists are aware of air flow status by observing how the GI tract expands or shrinks through the camera images by combining their finger movements to activate/deactivate insufflation.

There are two scenarios when insufflation control could not be performed properly: one scenario occurs when the insufflation valve gets faulty and air keeps leaking through the inactivated, malfunctioning valve. The other scenario occurs when insufflation is done more frequently than usual from both sides without each user recognizing it. Although the individual user may insufflate no more than usual, the total amount by the multiple users may exceed the average amount by a single user in conventional endoscopy.

In typical endoscopic systems, the valve control unit is connected to an insufflator that keeps feeding air, so the insufflation valve in the valve control unit needs to be closed by default to avoid unnecessary air feeding. When the activation button either on the endoscope or on the remote console is pressed, it sends a command through a valve controller to the valve to open it so that air goes into the patient body. When the button is not pressed, the valve is closed and no air should be fed into the patient body. If the insufflation valve gets faulty or broken and air is constantly pumped through the valve, undesired air flow goes into the patient body while the user does not notice it. This potentially causes the over-inflation problem.

In the latter case, if the activation buttons on both sides are controlled independently by a different user at the same time, there is a possibility that the total amount of air fed into the patient body could be more than how it is done with the conventional endoscope system, and hence this over-inflation could harm the patient.

WO2006013796 discloses a plug body of an endoscopic system as shown in Figure 20. A standard inlet seal as shown for conventional endoscope tools such as forceps which is one body made by a rubber. The body consists of two seals, one is for small endoscopy tools, and another is for large endoscopy tools. And the body also consists of a lip seal has one seal. Inlet seal is fitted onto a channel inlet with its elasticity.

An Endoscope Submucosal Dissection (ESD) procedure with robot assisted surgical endoscope system consists of three steps; insertion, ESD, and extraction. Insertion means that endoscope without surgical instruments gets close to tumor, which the tumor location has been discovered at prior screening. After that, surgical instruments are inserted into surgical instrument channel of endoscope and ESD is performed. The surgical instrument grasper grasps resected tissue and pulls the tissue away with the endoscope, after the tumor has been dissected.

The inlet seal is mounted onto surgical instrument channel inlet and it is necessary for the inlet seal to keep airtightness with and without a surgical instrument. On the other hand, even though the surgical instrument does not require much effort to be pulled away from a surgical instrument channel during ESD, it is of no inconvenience to the endoscopist and operator if the inlet seal can keep airtightness with the surgical instrument in the step of ESD and extraction.

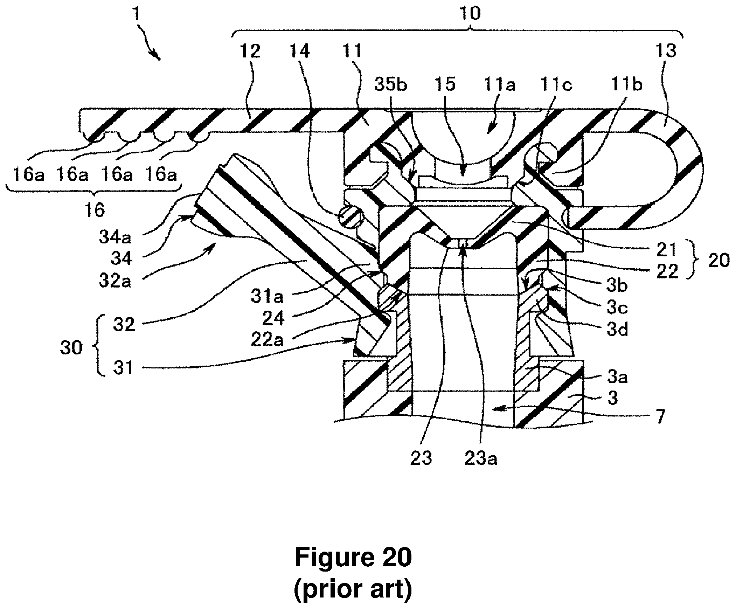

The standard inlet seal for conventional endoscopy tools is made from rubber and consists of a seal (see #23 of Figure 20) for endoscopy tools having a small diameter. It is used for airtightness without endoscopy tools, its shape returns to original shape and it also keeps airtightness after the endoscope tool is removed. An alternative seal (see #15 of Figure 20) for endoscopy tools may have a large diameter, is sometimes a part of lip seal (see #10 of Figure 20) and is mounted onto a channel inlet on the control body by its elasticity. In the case that endoscopy tools are somewhat larger than the seal, the lip seal is removed but its friction is increased significantly and the inlet seal is sometimes pulled away with endoscopy tool extraction. The surgical instruments are translated by one or more actuators such as motor, so it is preferable that friction between the inlet seal and the surgical instrument is low.

As a consequence, there is a need for methods and systems which overcome the deficiencies of conventional endoscopy systems and to address at least some of the above problems or provide a useful alternative. More specifically, a need exists to provide rotational control of the instrument within the endoscope in order to determine the orientation of the instrument's distal end vis-à-vis the endoscope vision system for appropriate operational rotational control. It is also required to provide an inlet seal which is designed to make friction less and cost low

### SUMMARY OF THE INVENTION

According to a first aspect of the present invention, there is provided an insufflation control device for an endoscope system, comprising a master valve that is disposed downstream relative to an insufflator of the endoscope system and upstream relative to an endoscope of the endoscope system; a slave valve that is disposed downstream relative to the insufflator and upstream relative to the endoscope; and a valve controller that is configured to operationally control one or more of the master valve or the slave valve in response to commands received either from the endoscope system or from a remote console, wherein the master valve and the slave valve are disposed in series with each other.

In an embodiment, the valve controller is configured to operationally control both the master valve and the slave valve in response to the commands received either from the endoscope system or from the remote console, such that under normal operating conditions both the master valve and the slave valve are open or closed at substantially the same time.

In an embodiment, the valve controller is configured to operationally control only the master valve in response to the commands received either from the endoscope system or from the remote console, wherein under the normal operating conditions the slave valve is open and wherein the valve controller is configured to close the slave valve in response to an override command.

In an embodiment, the device further comprises an additional master valve that is disposed downstream relative to the insufflator, upstream relative to the endoscope, and in parallel with the master valve, and wherein the valve controller is further configured to operationally control the master valve, the slave valve and the additional master valve in response to the commands received either from the endoscope system or from the remote console, such that under the normal operating conditions the master valve, the slave valve and the additional master valve are open or closed at substantially the same time.

In an embodiment, the valve controller is configured to operationally control only the master valve and the additional master valve in response to the commands received either from the endoscope system or from the remote console, wherein under the normal operating conditions the slave valve is open and wherein the valve controller is configured to close the slave valve in response to an override command.

In an embodiment, the device further comprises an air flow sensor disposed in series with the master valve, downstream relative to the master valve and upstream relative to the endoscope, wherein the air flow sensor and the master valve are in fluid communication with an insufflation tube of the endoscope system, and wherein the air flow sensor is configured to provide an indication of air flow through the insufflation tube.

In an embodiment, the device further comprises a valve control unit, the valve control unit with the air flow sensor is further configured to provide an alert on a condition that the air flow through the insufflation tube that is determined by the air flow sensor is above a pre-determined level, which implies air leakage from the valve.

According to a second aspect of the present invention, there is provided an insufflation control device for an endoscope system, comprising a valve that is disposed downstream relative to an insufflator of the endoscope system and upstream relative to an endoscope of the endoscope system; a valve controller that is configured to operationally control the valve in response to commands received from the endoscope system, and/or configured to operationally control the valve in response to commands received from a remote console on a condition that a switch command is received at the valve controller.

In an embodiment, the switch command is transmitted from an input module associated with the endoscope system to the valve controller in response to a user instruction to switch control from the endoscope system to the remote console and vice versa.

In an embodiment, the switch command is transmitted from an input module associated with the remote console to the valve controller in response to a user instruction to switch control from the endoscope system to the remote console and vice versa.

According to a third aspect of the present invention, there is provided an insufflation control device for an endoscope system, comprising: a valve that is disposed downstream relative to an insufflator of the endoscope system and upstream relative to an endoscope of the endoscope system; a valve controller that is configured to operationally control the valve either in response to commands received from the endoscope system or from a remote console on a condition that an interlock command is received at the valve controller.

In an embodiment, the interlock command is transmitted from a docking module of the endoscope system to the valve controller based on a docking status of the endoscope.

In an embodiment, the device further comprises an output module that is in communication with (i) the valve, (ii) the input module associated with the endoscope system, and/or (iii) the input module associated with the remote console, wherein the output module is configured to provide an indication of: an operational status of the valve; a presence of the command received from the endoscope system; and/or a presence of the command received from the remote console.

In an embodiment, the device further comprises an air flow sensor disposed in series with the valve, downstream relative to the valve and upstream relative to the endoscope, wherein the air flow sensor and the master valve are in fluid communication with an insufflation tube of the endoscope system, and wherein the air flow sensor is configured to transmit a close command to the valve controller on a condition that the air flow through the insufflation tube that is determined by the air flow sensor is above a pre-determined level or is present for longer than a predetermined time.

According to a fourth aspect of the present invention, there is provided an endoscopy surgical instrument inlet device for an endoscope system comprising: a body having a channel extending through the thickness of the body, the body comprising a locking structure having an engagement portion that is correspondingly shaped to interlock against a fixture located at a proximate end of an endoscopy surgical instrument inlet so as to secure the endoscopy surgical instrument inlet device to the endoscopy surgical instrument inlet.

In an embodiment, the engagement portion comprises any one or more of a protrusion and a groove.

In an embodiment, the locking structure comprises a snap fit or a screw thread arrangement.

In an embodiment, the snap fit arrangement comprises at least one arm coupled to an outer surface of the body through a flexibly resilient member.

In an embodiment, the at least one arm extends past a distal end of the body that engages the proximate end of the endoscopy surgical instrument inlet.

In an embodiment, the screw thread arrangement is disposed along a portion of an inner wall of the channel extending through the thickness of the body.

In an embodiment, the device further comprises a seal disposed on a proximate end of the body, wherein the seal comprises a perforable portion which covers the channel extending through the thickness of the body.

In an embodiment, the perforable portion comprises at least one line of weakness establishing a tear portion retained by the seal after the perforable portion perforates along the at least one line of weakness.

In an embodiment, each of the line of weakness has open ends.

In an embodiment, the at least one line of weakness is disposed to form shapes that resemble any one of the following letters: C, H, N, X and I.

In an embodiment, the seal is manufactured from material having a smooth texture.

In an embodiment, the material comprises any one or more of plastic, paper, resin, rubber and gel.

In an embodiment, the body further comprises a seal arrangement disposed downstream of the seal disposed on the proximate end of the body.

In an embodiment, the seal arrangement comprises an inner seal disposed along an inner wall of the channel extending through the thickness of the body, the inner seal having a hole aligned with a centre of the perforable portion of the seal disposed on the proximate end of the body.

In an embodiment, the seal arrangement further comprises a seal disposed on a distal end of the body, the seal adapted to contact with an endoscopy surgical instrument inlet over which the endoscopy surgical instrument inlet device is placed when in use.

In an embodiment, the seal disposed on the distal end of the body and the inner seal are integrated into one part.

In an embodiment, the seal disposed on the proximate end of the body and the seal arrangement are coated with lubricant.

In an embodiment, an endoscopy surgical instrument inlet cover may comprise a plurality of endoscopy surgical instrument inlet devices, wherein each of the endoscopy surgical instrument inlet devices is positioned to have the channel of its body aligned with a respective endoscopy surgical instrument inlet over which the endoscopy surgical instrument inlet cover is placed when in use.

According to a fifth aspect of the present invention, there is provided an endoscopy surgical instrument inlet device comprising: a body having a channel extending through the thickness of the body and a seal disposed on a proximate end of the body, wherein the seal comprises a perforable portion which covers the channel extending through the thickness of the body.

In an embodiment, the perforable portion comprises at least one line of weakness establishing a tear portion retained by the seal after the perforable portion perforates along the at least one line of weakness.

In an embodiment, the body comprises a locking structure having an engagement portion that is correspondingly shaped to interlock against a fixture located at a proximate end of an endoscopy surgical instrument inlet so as to secure the endoscopy surgical instrument inlet device to the endoscopy surgical instrument inlet.

In an embodiment, the engagement portion comprises any one or more of a protrusion and a groove.

In an embodiment, the locking structure comprises a snap fit or a screw thread arrangement.

In an embodiment, the snap fit arrangement comprises at least one arm coupled to an outer surface of the body through a flexibly resilient member.

In an embodiment, the at least one arm extends past a distal end of the body that engages the proximate end of the endoscopy surgical instrument inlet.

In an embodiment, the screw thread arrangement is disposed along a portion of an inner wall of the channel extending through the thickness of the body.

In an embodiment, an endoscopy surgical instrument inlet cover may comprise a plurality of endoscopy surgical instrument inlet devices as disclosed, wherein each of the endoscopy surgical instrument inlet devices is positioned to have the channel of its body aligned with a respective endoscopy surgical instrument inlet over which the endoscopy surgical instrument inlet cover is placed when in use.

### BRIEF DESCRIPTION OF THE DRAWINGS

Example embodiments of the invention will be better understood and readily apparent to one of ordinary skill in the art from the following written description, by way of example only, and in conjunction with the drawings. The drawings are not necessarily to scale, emphasis instead generally being placed upon illustrating the principles of the invention, in which:
Figure 1 shows a cross sectional illustration 100 of a distal end of an instrument in an endoscopic system according to an example embodiment.
Figure 2A shows a schematic illustration of an optical emitter in an endoscope according to an example embodiment.
Figure 2B shows a schematic illustration of an alternative embodiment of the optical emitter of Figure 2A.
Figures 3A to 3C show schematic diagrams of the distal end of an instrument according to an example embodiment.
Figure 4 shows a schematic illustration of the instrument of Figure 3A to 3C according to an example embodiment.
Figure 5 shows a schematic illustration 500 of the instrument of Figure 4 according to an example embodiment.
Figure 6 shows a cross sectional view 600 of the instrument of Figure 4 according to an example embodiment.
Figure 7 shows a schematic diagram 700 illustrating a rotation hampering device of an instrument according to an example embodiment.
Figure 8 shows a schematic diagram 800 illustrating a side view of a rotation hampering device in the detachable hood of Figure 4 according to an example embodiment.
Figures 9A to 9D show schematic diagrams 900 of an engageable device of the instrument of Figure 4 according to an example embodiment.
Figure 10A shows a schematic diagram 1000 of a typical simple structure of a valve control unit in an endoscopic system while Figure 10B shows a graphic representation of the valve signals and status of the valve control unit of Figure 10A according to an example embodiment.
Figure 11A shows a schematic diagram 1100 of two synchronized valves in series in a valve control unit in an endoscopic system while Figure 11B shows a graphic representation of the valve signals and status of the valve control unit of Figure 11A according to an example embodiment.
Figure 12A shows a schematic diagram 1200 of two non-synchronized valves in series in a valve control unit in an endoscopic system while Figure 12B shows a graphic representation of the valve signals and status of the valve control unit of Figure 12A according to an example embodiment.
Figure 13A shows a schematic diagram 1300 of multiple valves in parallel and series in a valve control unit of an endoscopic system while Figure 13B shows a graphic representation of the valve signals and status of the valve control unit of Figure 13A according to an example embodiment.
Figure 14A shows a schematic diagram 1400 of multiple valves in parallel and series in a valve control unit of an endoscopic system while Figure 14B shows a graphic representation of the valve signals and status of the valve control unit of Figure 14A according to an example embodiment.
Figure 15A shows a schematic diagram 1500 of a valve control unit having a flow sensor in an endoscopic system while Figure 15B shows a graphic representation of the valve signals and status of the valve control unit of Figure 15A according to an example embodiment.
Figure 16A shows a schematic diagram 1600 of a valve control unit having a flow sensor and two valves in an endoscopic system while Figure 16B shows a graphic representation of the valve signals and status of the valve control unit of Figure 16A according to an example embodiment.
Figure 17 shows a schematic diagram 1700 illustrating user-controllable valve activation settings in an endoscopic system according to an example embodiment.
Figure 18 shows a schematic diagram 1800 illustrating visual and/or audio indicators in an endoscopic system according to an example embodiment.
Figure 19A shows a schematic diagram 1900 illustrating a suction button having in an endoscopic system while Figure 19B shows a graphic representation of the valve signals and the amount of air in the endoscopic system of Figure 19A according to an example embodiment.
Figure 20 is a prior art showing a cross sectional diagram of a plug body in an endoscope.
Figure 21A shows a cross section of an inlet seal locking structure in an endoscopic system while Figure 21B shows a perspective view of the inlet seal locking structure of Figure 21A.
Figure 22A shows a cross section of an inlet seal locking structure having a screw in an endoscopic system while Figure 22B shows a perspective view of the inlet seal locking structure of Figure 22A according to an example embodiment.
Figure 22C shows perspective views of the guiding structure of Figure 22B according to an example embodiment.
Figure 23A shows a perspective view of an exterior of the inlet seal in an endoscopic system while Figure 23B shows a cross section of the inlet seal of Figure 23A according to an example embodiment.
Figure 24 shows perspective views of an inlet seal on an endoscope having a plurality of surgical instrument channels according to an example embodiment

### DETAILED DESCRIPTION

In the following description, an instrument may be defined as any tool which is inserted through an endoscope for any purpose. For example, the instrument can be a tube to provide water or air or other fluid to a target site. Alternatively, an instrument may be used to take a biopsy at a target site or remove a polyp or other growth at the target site.

In accordance with present embodiments, an endoscopic system is described which includes an endoscope and an instrument. The endoscope has a hollow tube formed therein for inserting the instrument therethrough. The hollow tube may be referred to as a "lumen". The instrument has a first end for operational control and a second distal end for instrument operation at a distal end of the endoscope. The distal end of the endoscope has an illumination device and a vision device for providing illuminated vision at the distal end of the endoscope for operational control of the instrument operation at the distal end of the instrument. The distal end of the instrument and the distal end of the endoscope are interoperably coupled for rotational control of the instrument with respect to the endoscope.

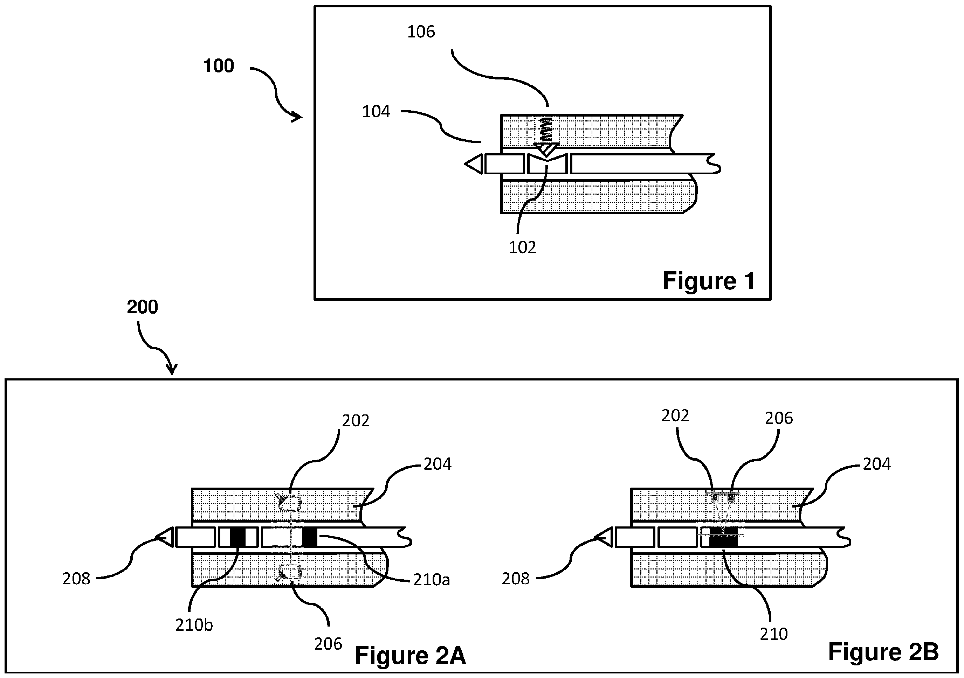

Figure 1 shows a cross sectional illustration 100 of a distal end of an instrument in an endoscopic system according to an example embodiment. As shown in the Figure, a divot 102 is formed at a predetermined distance from the distal end 104 of the instrument and a compliant nipple 106 is located in the endoscope (not shown in the Figure) at the predetermined distance from the distal end 104 of the endoscope, the nipple 106 being a projecting structure conversely shaped to the divot 102. It can be appreciated that multiple embodiments of the nipple 106 are possible. For example, figure 1 shows one preferred embodiment of the nipple 106 utilizing a spring. In accordance with this embodiment, the divot 102 and the spring-loaded nipple 106 interoperably couple together when the instrument and the endoscope are oriented in a predetermined manner for mechanical prevention of further rotation of the instrument with respect to the endoscope. Compliance of the nipple 106 is advantageous as it may allow the instrument to be introduced in any orientation including orientations other than the predetermined orientation. The interoperable coupling between the divot 102 and the nipple 106 can occur when the instrument is subsequently brought into the predetermined orientation.

On the other hand, it can be appreciated that compliance of the divot 102 can be an equally acceptable method for achieving rotation prevention when the said instrument is introduced in any orientation. For example, the divot 102 may be one that is shaped as a protruding structure in order to be interoperably coupled with the nipple 106 which may be a recess positioned on the endoscope. It should also be appreciated that the position of the nipple 106 and the divot 102 may be reversed, such that the nipple 106 is located on the instrument and the divot 102 is located on the endoscope.

For purposes of endoscope reprocessing and future reuse, it is advantageous that the interior surface of the endoscope lumen be smooth to aid in effective cleaning. In order to achieve such an objective, compliance devices such as springs may be sealed inside the endoscope wherein the nipple 106 is formed, such that the compliance device presses radially inwards on a flexible portion of the lumen.

In a second embodiment, the endoscope comprises an optical sensor system located a predetermined distance from the distal end of the endoscope. The optical sensor system includes an optical emitter, an optical receiver and an optical interactive device that is located on in the instrument or in the instrument at the predetermined distance from the distal end of the instrument. In accordance with this second embodiment, the optical sensor system and the optical interactive device operate together to generate an orientation signal indicating an orientation of the instrument with respect to the endoscope.

Figure 2A shows a schematic illustration 200 of an optical emitter in an endoscope according to an example embodiment. In the Figure, the optical emitter 202 is located within the endoscope 204 to emit light of a predetermined frequency or frequencies from a first internal side and the optical receiver 206 is located within the endoscope 204 to receive light of the predetermined frequency or frequencies at a second side of the endoscope 204 opposite the first side. The optical interactive device 208 in accordance with this embodiment includes a partially transparent portion 210a or a fully transparent portion 210b of the instrument. The optical sensor system operates with the optical interactive device 208 to generate an orientation signal indicating an orientation of the instrument with respect to the endoscope 204 when the light from the optical emitter 202 passes through the partially transparent portion 210a or the fully transparent portion 210b of the instrument and is received at the optical receiver 206.

It should be appreciated that the optical interactive device 208 may transmit less light than other portions of the instrument, rather than transmitting more light, as the modification of the transmitted light intensity could also be used to generate an orientation signal indicating an orientation of the instrument with respect to the endoscope 204. It should also be appreciated that a second alternate embodiment may include the optical interactive device 208 having a similar total absorption to other portions of the instrument, except that the incident light on the optical interactive device 208 may be diffused or reflected away from the optical receiver 206 by means of an optical diffuser, mirrors, or a light pipe. It should be further appreciated that a further alternate embodiment exists, wherein the optical interactive element imparts a preferential polarization direction to the transmitted light that is detectable by the optical receiver 206 by means of a parallel or cross-polarized filter to the preferred polarization direction imparted by the optical interactive element.

Figure 2B shows a schematic illustration of an alternative embodiment of the optical emitter of Figure 2A. In the Figure, the optical emitter 202 and the optical receiver 206 are located within the endoscope 204 to emit light of a predetermined frequency or frequencies from a first internal side and receive light of the predetermined frequency or frequencies at the first internal side of the endoscope 204. In accordance with this embodiment, the optical interactive device 208 comprises a reflective device 210 on the instrument and the optical sensor system operates with the optical interactive device 208 to generate an orientation signal indicating an orientation of the instrument with respect to the endoscope 204 when the light from the optical emitter 202 reflects off the reflective device 210 on the instrument and is received at the optical receiver 206.

It can be appreciated that the optical interactive device 208 may alternatively be less reflective than the remainder of the instrument, rather than more reflective, as this modification of the reflected light could also be used to generate an orientation signal indicating an orientation of the instrument with respect to the endosocope 204. It should also be appreciated that a further alternate embodiment exists wherein the optical interactive device 208 may have a similar total reflectivity to other portions of the instrument, except that the light is reflected away from the optical receiver 206 by means of a diffuse reflective surface. It should be further appreciated that the optical interactive element may impart a preferential polarization direction to the reflected light that is detectable by the optical receiver 206 by means of a parallel or cross-polarized filter to the preferred polarization direction imparted by the optical interactive element.

For purposes of endoscope reprocessing and future reuse, it is advantageous that the optical emitter 202 and the optical receiver 206 are able to withstand the cleaning and sterilization chemical exposure without detrimental effects to their functions. As such, it is advantageous that the optical emitter 202 and optical receiver 206 of the embodiments shown in Figures 2A and 2B be sealed inside the endoscope 204 with clear or translucent elements at the first and / or second internal surfaces to allow light to pass through the sealed enclosure of the endoscope 204.

In an embodiment, the endoscope may include a magnetic field sensing system located a predetermined distance from the distal end of the endoscope 204. The magnetic field sensing system may include a magnetic field sensor and a magnetic field producer that is located on or in the instrument the predetermined distance from the distal end of the instrument. In this embodiment, the magnetic field sensor and the magnetic field producer operate together to generate an orientation signal indicating an orientation of the instrument with respect to the endoscope 204. The magnetic field sensor may comprise one or more of the following: a hall-effect sensor or a magnetometer. The magnetic field producer may comprise one or more of the following: a permanent magnet or a coil of wire that is operably energizable to produce a magnetic field. The magnetic field producer may be aligned on or within the instrument such that a change of orientation of the instrument with respect to the endoscope will result in a detectible change of magnetic field strength or magnetic field direction at the magnetic field sensor. As those skilled in the art will realize, while we have described the magnetic field sensor in the endoscope 204 and the magnetic field producer located on or in the instrument, the order could be reversed. That said placing the magnetic field sensor in the endoscope 204 has its advantages: (1) the magnetic field sensor requires more space while the magnetic field producer can fit better in the constrained volume of the instrument; (2) the magnetic field sensor is more expensive than the magnetic field producer and so placing the magnetic field sensor in the endoscope 204, a reusable capital piece of equipment makes more sense than placing the magnetic field sensor in the instrument which is a limited use device which gets one to several uses before being disposed; and (3) the magnetic field sensor requires multiple wiring such as ground, power and data wires which are more easily and less costly addressed in the reusable capital piece of equipment (i.e., the endoscope) than in the disposable instrument.

In accordance with further embodiments, the endoscope 204 may include a detachable hood at the second distal end of the endoscope 204 and the distal end of the instrument and the detachable hood are interoperably coupled for rotational control of the instrument with respect to the endoscope 204. A detachable hood holds an advantage over embodiments where the hood is permanently incorporated into the endoscope 204 where the components of the endoscopic system may encounter degradation of function over the life of the endoscope 204. In such cases, a detachable hood allows restoration of the components' original function, without replacement of the entire endoscope 204. Further, the endoscopic system may include features that are difficult to clean due to restricted cleaning access, such as small internal features capable of trapping contaminants or incompatibility of the endoscopic system with one or more elements of the cleaning process. In such cases, a single use detachable hood can ensure a clean condition for each use, while also eliminating the design constraints associated with cleaning. Several guide rings forming a guide ditch on the flexible shaft of the surgical instrument are located up to a predetermined distance from the distal end. As the guide ring consists of rigid components, a predetermined distance must be allocated between each guide ring in order to maintain flexibility of the shaft.

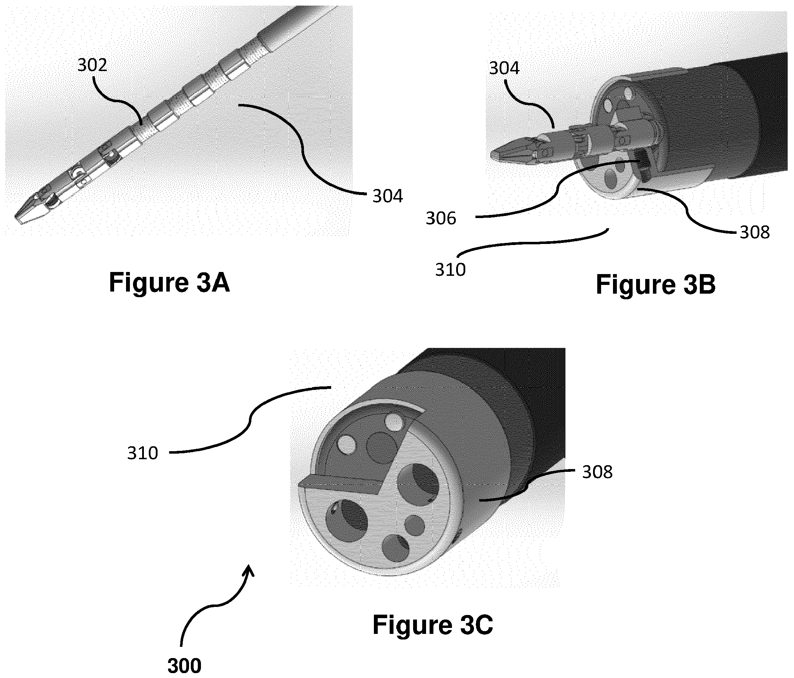

Figures 3A to 3C show schematic diagrams 300 of the distal end of an instrument according to an example embodiment. In the Figures, a ditch 302 is formed in the instrument 304 at a predetermined distance from the distal end of the instrument and a spring pin 306 is located in the detachable hood 308 at the predetermined distance from the distal end of the detachable hood 308. In this manner, the spring pin 306 and the ditch 302 interoperably engage when the instrument 304 and the endoscope 310 are oriented in a predetermined manner for mechanical prevention of further rotation of the instrument 304 with respect to the endoscope 310. While other compliant structures can be used in accordance with this embodiment, a key advantage of the embodiment as depicted in Figures. 3A to 3C is that spring pins 306 occupy a relatively small space inside the detachable hood 308, as compared to other structures. This is especially important for maintaining a small size of the endoscope 310 and the endoscope hood 308, such that insertion into the body is not impeded and such that the patient comfort is maximized.

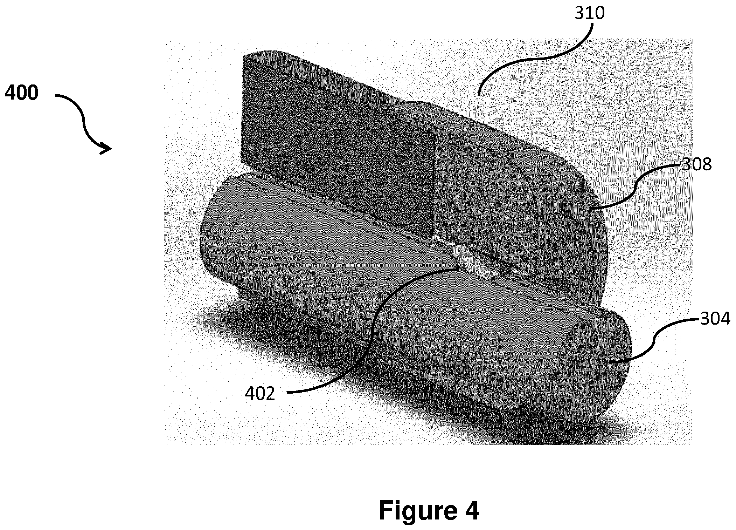

Figure 4 shows a schematic illustration 400 of an instrument of Figures 3A to 3C according to an example embodiment. As shown in the Figure, a leaf spring 402 is located on the instrument 304 a predetermined distance from the distal end of the instrument 304 and a ditch (not shown in the figure) is formed in the detachable hood 308 at the predetermined distance from the distal end of the detachable hood 308. The leaf spring 402 and the ditch interoperably engage when the instrument 308 and the endoscope 310 are oriented in a predetermined manner for mechanical prevention of further rotation of the instrument 304 with respect to the endoscope 310. A key advantage of the embodiment depicted in Figure 4 is that the compliant aspect of the system consists of a single element which serves to minimize cost. In other words, the detachable hood 308 and the leaf spring 402 in this embodiment may be a single use element, and therefore cost may be reduced in order to minimize the per procedure cost to the patient, the hospital, and society at large.

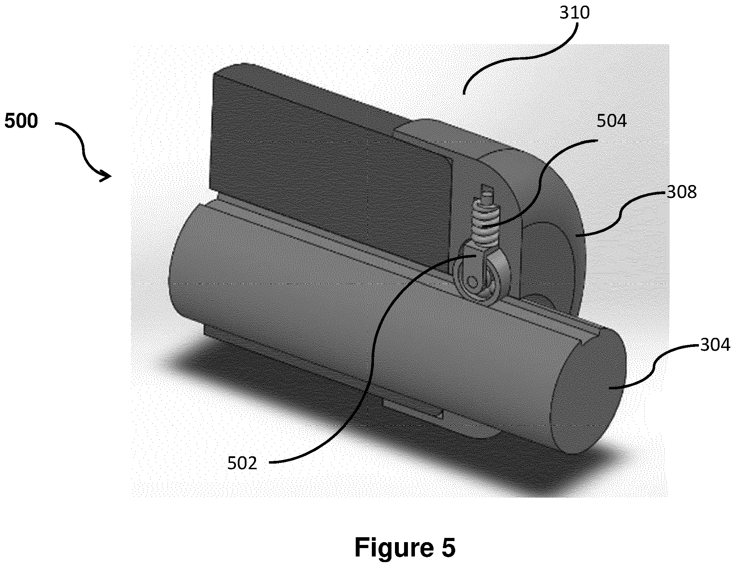

Figure 5 shows a schematic illustration 500 of an instrument of Figure 4 according to an example embodiment. In the Figure, a ditch is formed in the instrument 304 at a predetermined distance from the distal end of the instrument 304 and a compliant rolling element 502 is located on the detachable hood 308 at the predetermined distance from the distal end of the detachable hood 308 such that at least one compliant rolling element 502 and the ditch interoperably engage when the instrument 304 and the endoscope 310 are oriented in a predetermined manner for mechanical prevention of further rotation of the instrument 304 with respect to the endoscope 310. The compliant rolling element 502 is configured to roll with minimal friction when the instrument 304 is translated in the axial direction, while imparting reaction forces on the ditch that prevent rotation of the instrument 304 relative to the endoscope 310. Possible embodiments of the rolling element 502 include but are not limited to bearings or bushings.

In addition, compliance of the rolling element 502 may be configured in multiple ways. One preferred embodiment of compliance involves a resilient material disposed symmetrically about the outer diameter of the rolling element 502 at the contact point between the rolling element 502 and the ditch, which can compress when the instrument 304 is inserted in orientations other than the predetermined orientation. A second preferred embodiment of rolling element 502 compliance involves adding with compliance to the axle on which the rolling element 502 rotates. Figure 5 shows such a preferred embodiment of compliance wherein the axle of the rolling element 502 is spring-loaded 504 to press the rolling element 502 into the ditch when the instrument 304 reaches the predetermined orientation relative to the endoscope 310.

The embodiment shown in Figure 5 may have the advantage of having the lowest friction in the translation direction. This advantage may be important in applications where translation movement are to be precise, where case stick-slip behaviour of static friction from other embodiments may cause uncontrolled translation movements of the instrument 304. A lower translation friction may also be an advantage in applications where available instrument translation force is limited.

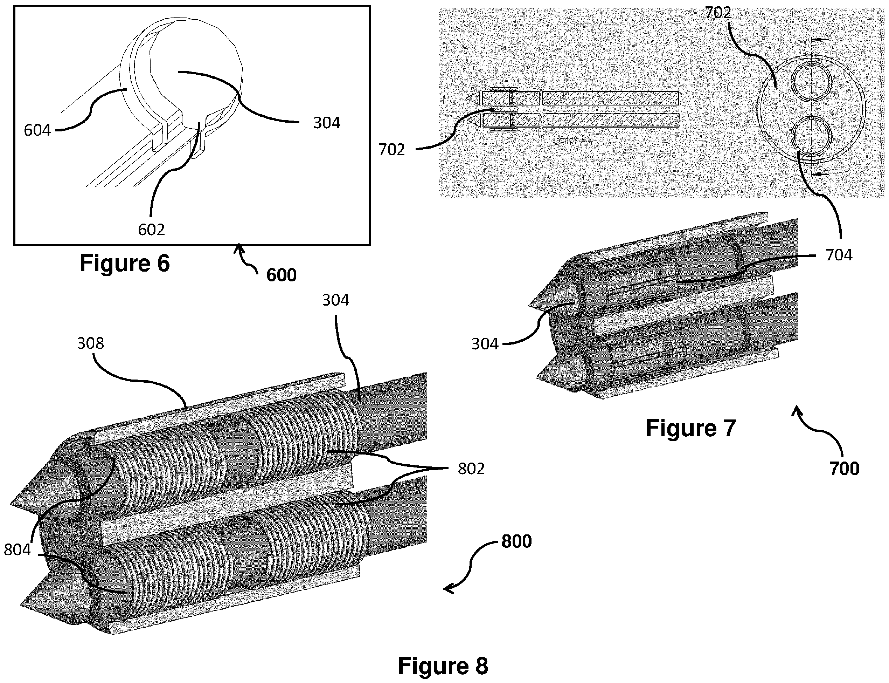

Figure 6 shows a cross sectional view 600 of an instrument of Figure 4 according to an example embodiment. In the Figure, a bump 602 is formed on the instrument 304 at a predetermined distance from the distal end of the instrument 304 and a C-spring 604 is located in the detachable hood the predetermined distance from the distal end of the detachable hood (not shown in the Figure) such that the bump 602 and the C-spring 604 are interoperably engaged when the instrument 304 and the endoscope (not shown in the Figure) are oriented in a predetermined manner for mechanical prevention of further rotation of the instrument 304 with respect to the endoscope.

The embodiment shown in Figure 6 shares the single compliant element cost advantage of the embodiment as shown in Figure 4. More specifically, the embodiment of Figure 6 may have the additional advantage of predictable rotational torque limitation. If the instrument 304 experiences torsion away from the predetermined orientation, the C-spring 604 will provide a restoring torque up to a predictable limit, depending on the bending stiffness of the C-spring 604. Above this restoration torque limit, the bump 602 may disengage from the C-spring 604, and the instrument 304 can assume an orientation other than the predetermined orientation. This can be a key safety advantage if the instrument 304 is likely to experience large rotational torques that could damage other components of the endoscopic system. The predictability of the limit torque at which the bump 602 disengages from the C-spring 604 may advantageously ensure that disengagement remains within both minimum and maximum torsion limits.

In a further embodiment, a rotation hampering device may be located in the detachable hood 308 (as shown in Figures 4 and 5). The rotation hampering device may impart high friction against the instrument 304 when the instrument 304 attempts to move rotationally within the rotation hampering device while imparting less friction against the instrument 304 when the instrument 304 attempts to move translationally within the rotation hampering device for reduction of rotation of the instrument 304 with respect to the endoscope 310.

In the coulomb model of friction, friction is proportional to the normal force between the sliding surfaces. Even in less idealized models, friction is monotonically increasing with increasing normal force between the contacting surfaces. As such, an endoscope mounted device may be configured so as to increase or hold constant its radial normal force on the instrument shaft when the instrument is rotated, while holding constant or decreasing its radial normal force on the instrument when the instrument is translated. Such a device will then necessarily apply more friction in rotation prevention than in translation prevention. This concept is the fundamental principle behind the embodiments shown in Figures 7 and 8.

Figure 7 shows a schematic diagram 700 illustrating a rotation hampering device of an instrument according to an example embodiment. As shown in the Figure, the rotation hampering device 702 includes at least one elastomeric tube 704 with teeth disposed around the internal circumference of the elastomeric tube 704 such that the teeth contacts the instrument 304 at the centre of the tube 704 and such that a radial inward normal force is applied by the teeth onto the instrument 304. Translational and rotational movement of the instrument 304 results in translational and rotational deflection of the teeth, respectively. The teeth are configured to be more compliant in the translation direction than in the rotation direction. As such, the teeth maintain their radial normal force on the instrument 304 to a larger angle of deflection in the radial direction than in the translation direction. This allows for more frictional strain energy to be exerted on the instrument 304 in the rotation direction than in the translation direction, providing more resistance to rotation than translation. If a rotation torque applied to the instrument 304 exceeds the static friction limit of the elastomeric teeth, the instrument 304 will no longer hold in the predetermined orientation. However, such a frictional torque is preferentially increased compared to translational friction.

The embodiment as shown in Figure 7 may provide the advantage of holding the instrument 304 in any preferred orientation. A preferred holding orientation may be determined as either the orientation of the instrument when the instrument is initially inserted into the rotation hampering device 702, or the orientation of the instrument 304 when the applied instrument torque last exceeded the static friction limit of the rotation hampering device 702. In this manner, the preferred holding orientation is advantageously reconfigurable to suit clinical operation requirements.

Figure 8 shows a schematic diagram 800 illustrating a side view of a rotation hampering device in the detachable hood of Figure 4 according to an example embodiment. As shown in Figure 8, the rotation hampering device includes a left hand coil 802 located in the detachable hood 308 and a right hand coil 804 also located in the detachable hood 308. The left hand coil 802 and the right hand coil 804 function together as an anti-rotation clutch such that the left hand coil 802 imparts high friction against the instrument 304 by contracting radially when the instrument 304 attempts to rotate to the right and the right hand coil 804 imparts high friction against the instrument 304 by contracting radially when the instrument 304 attempts to rotate to the left. In addition, the left hand coil 802 and the right hand coil 804 impart less friction against the instrument 304 when the instrument 304 attempts to move translationally as neither coil contracts radially onto the instrument 304 during translational movement of the instrument 304. The embodiment as shown in Figure 8 may share the reconfigurable holding orientation advantage of the embodiment of Figure 7. In this manner the preferred holding orientation is reconfigurable to suit clinical operation requirements.

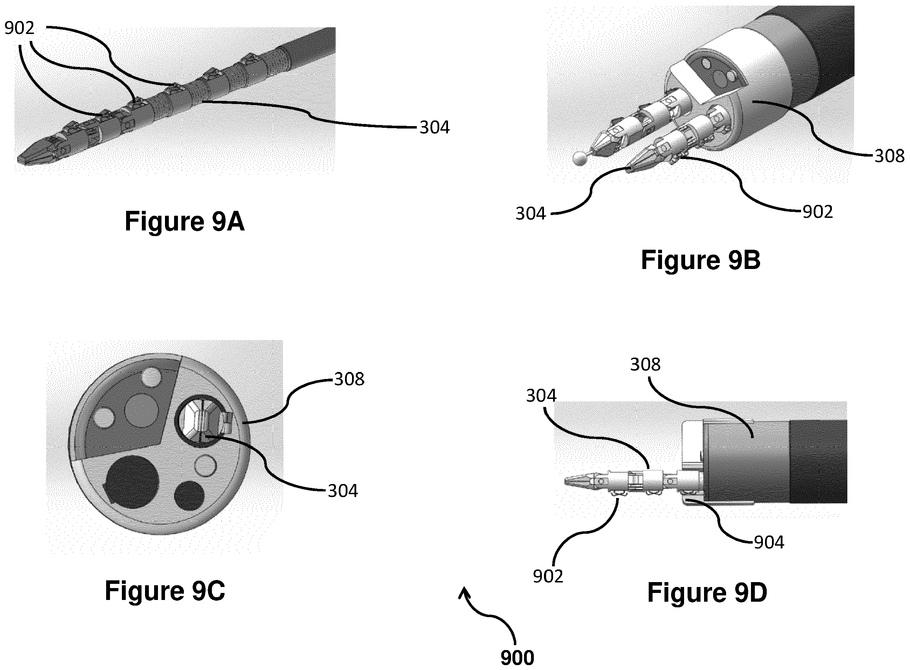

Figures 9A to 9D show schematic diagrams of an engageable device of an instrument according to an example embodiment. In the Figures, at least one engageable device 902 is formed on the instrument 304 a predetermined distance from the distal end of the instrument 304 and a corresponding structure 904 is located on the detachable hood 308 the predetermined distance from the distal end of the detachable hood 308. In this embodiment, the engageable device 902 and the corresponding structure 904 interoperably engage when the instrument 304 is extended through the endoscope a certain distance and oriented in a predetermined manner for mechanical prevention of further rotation of the instrument 304 with respect to the endoscope. Even though more space in the cross-section of the instrument 304 is required, the engageable device 902 may be fully retractable to within a circular instrument cross-section, thereby a round cross-section endoscope lumen may be utilized, which may offer easier cleaning than with non-circular endoscope lumen.

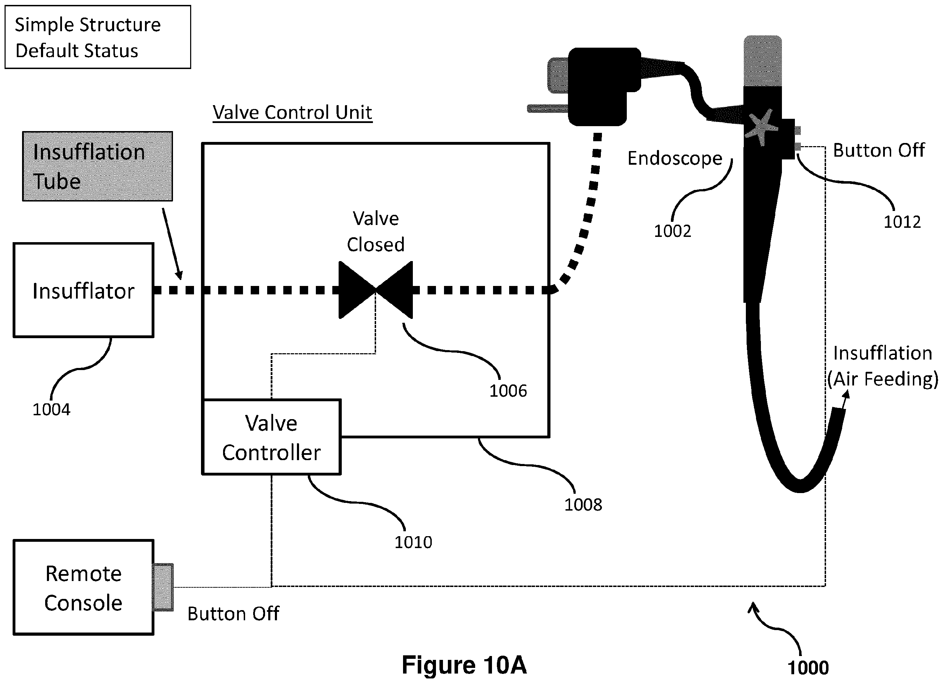

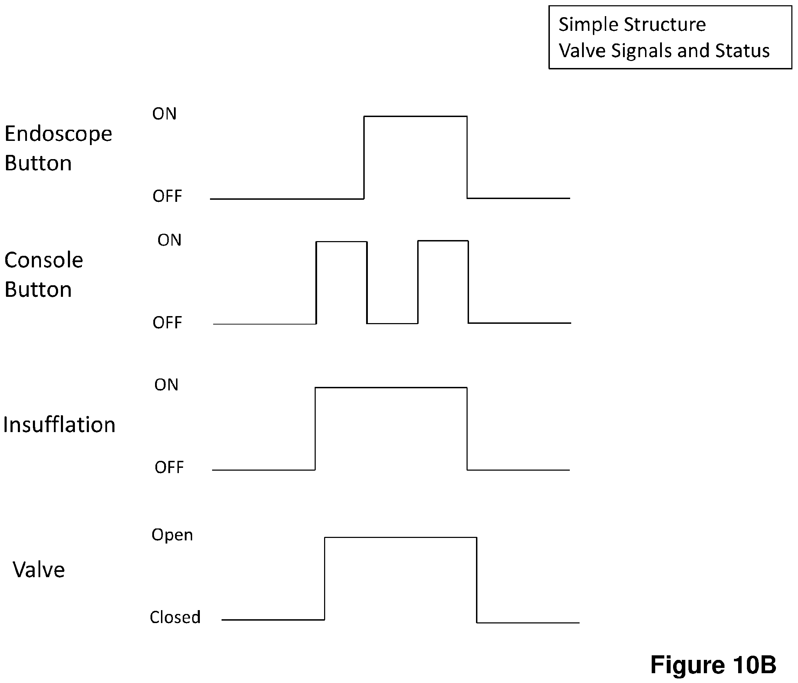

Figure 10A shows a schematic diagram 1000 of a typical simple structure of a valve control unit in an endoscopic system while Figure 10B shows a graphic representation of the valve signals and status of the valve control unit of Figure 10A according to an example embodiment. In the Figure, the endoscope 1002 may be connected to an insufflator 1004 via an insufflator valve 1006. The insufflator 1004 provides air to the endoscope 1002 during an endoscopy procedure to obtain a clear vision of a surgical site. As shown in the Figure, the valve control unit 1008 includes the insufflation valve 1006 and a valve controller 1010. The insufflation valve 1006 is closed by default to avoid unnecessary air feeding into the endoscope 1002. In this default state, the activation button 1012 located on the endoscope 1002 is not pressed and the insufflation valve 1006 is closed to prevent air from entering into the patient through the endoscope 1002. When the activation button 1012 is pressed, a command is sent to the valve controller 1010 which in turn commands the insufflation valve 1006 to open and air being fed into the patient through the endoscope 1002.

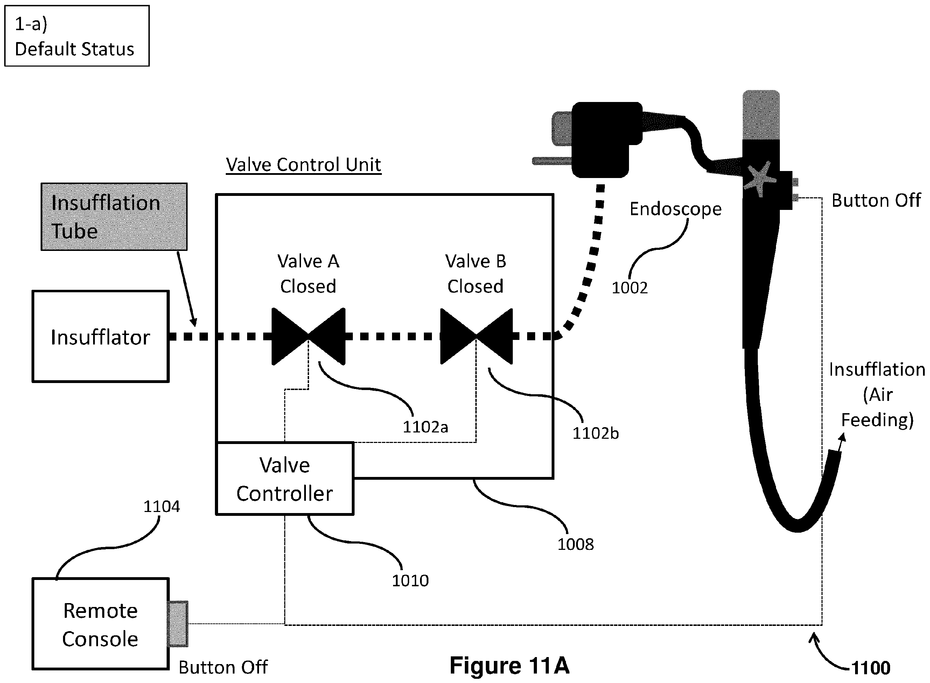

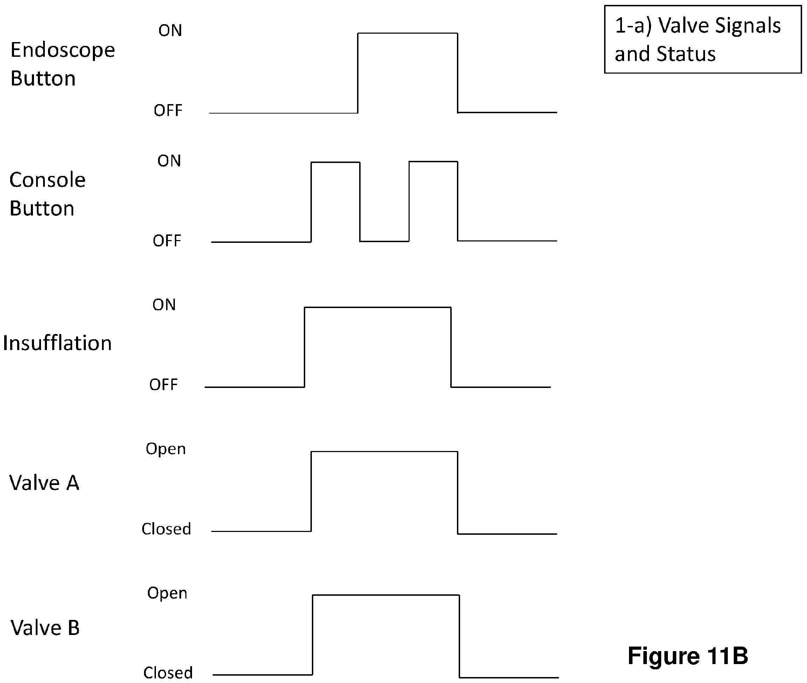

As previously described, improper insufflation control may be present when the insufflation valve 1006 of the valve control unit 1008 is faulty. In order to overcome such a problem, embodiments of the present invention provide multiple valves in the valve control unit 1008. An example embodiment of multiple valves is shown in Figure 11A, while Figure 11B shows the graphic representation of the valve signals and status of the valve control unit of Figure 11A according to an example embodiment. In Figure 11A, two valves 1102a 1102b are positioned in series and their motion are synchronized. As shown in the Figure, both valves 1102a 1102b are closed by default. Under normal conditions, the two valves 1102a 1102b are open and closed at the same time whenever they receive a command from the endoscope 1002 or from a remote console 1104. It can be understood that the remote console 1104 refers to a console that is remote from the endoscope 1002. When either valve 1102a 1102b malfunctions, there are two possible scenarios: (i) the faulty valve does not open or (ii) the faulty valve does not close. For the first possible scenario, there will be no air leakage, and there is no risk related to over-inflation posed to the patient. For the second scenario, as long as the other valve is not faulty, i.e., it opens and closes based on a command, the functioning valve works similarly as a one-valve system and thus over-inflation does not occur.

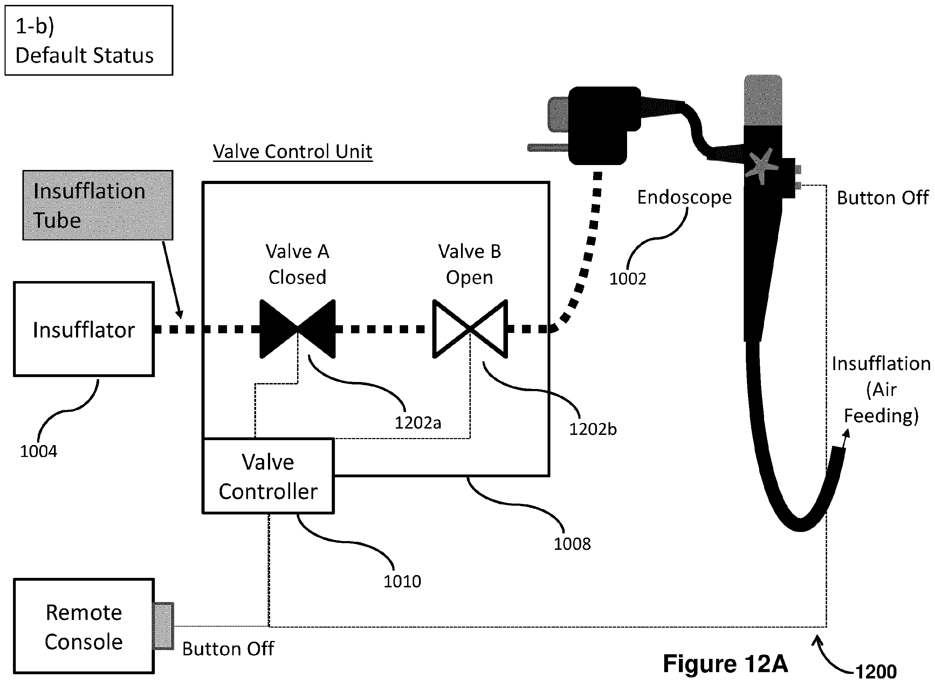

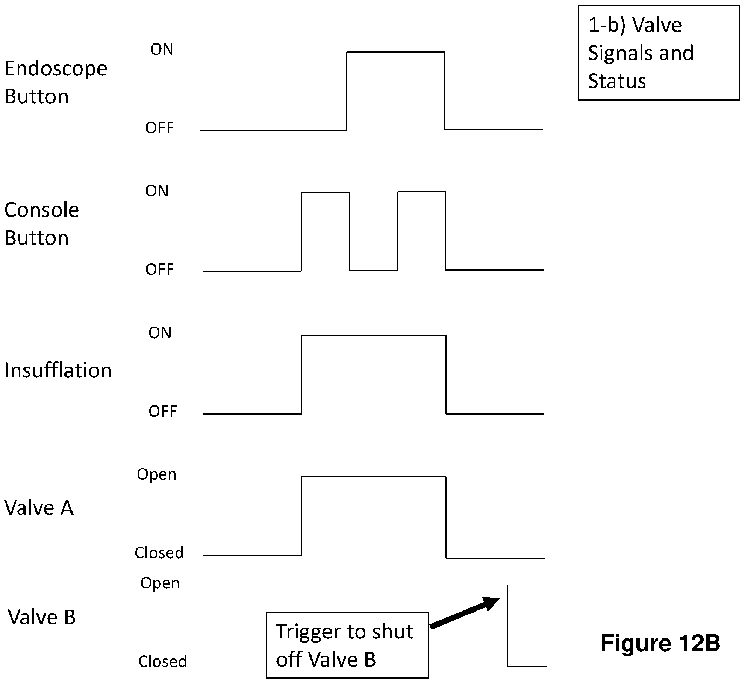

Figure 12A shows a schematic diagram 1200 of two non-synchronized valves in series in a valve control unit in an endoscopic system while Figure 12B shows a graphic representation of the valve signals and status of the valve control unit of Figure 12A according to an example embodiment. In the Figure, two valves 1202a 1202b are placed in series. Valve A 1202a is closed while the other valve B 1202b is open by default. In this embodiment, only valve A 1202a is controlled by the user in order to feed air. When an error is detected from valve A 1202a such that it malfunctions and cannot close, the air feeding can be shut off by closing valve B 1202b. It can be appreciated that the positioning of valves A 1202a and B 1202b can be reversed, i.e., either one valve can be placed near to the insufflator 1004 or near to the endoscope 1002, as long as they are not synchronized by default.

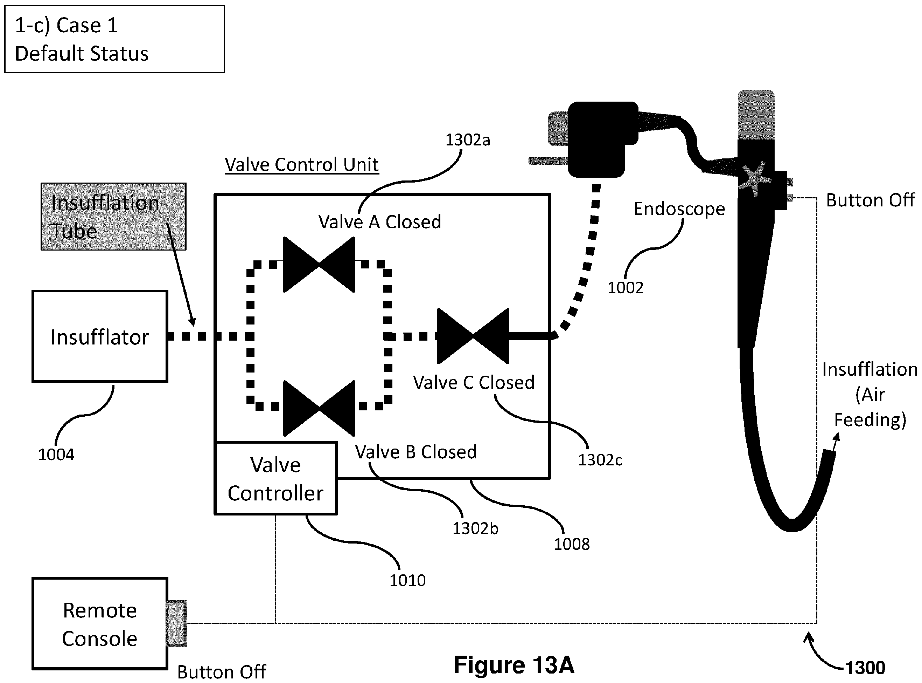

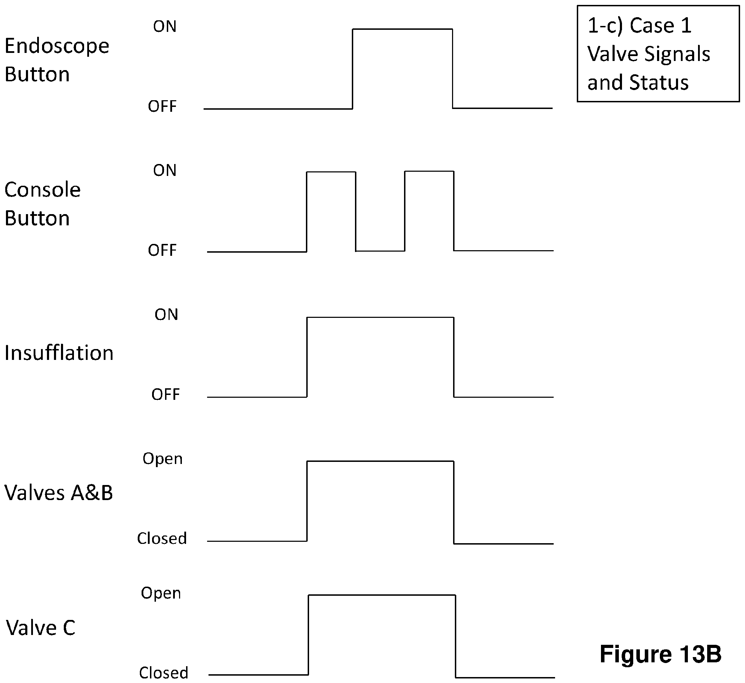

Figure 13A shows a schematic diagram 1300 of multiple valves in parallel and series in a valve control unit of an endoscopic system while Figure 13B shows a graphic representation of the valve signals and status of the valve control unit of Figure 13A according to an example embodiment. In the Figure, multiple valves 1302a 1302b 1302c are positioned in parallel and in series. For example and as shown, valve A 1302a and valve B 1302b are in parallel, and they are synchronized in motion. In other words, valves A 1302a and B 1302b open or close at the same time. A third valve C 1302c is connected to valve A 1302a and B 1302b in series. Valve C 1302c works similarly to the valves shown in Figures 11A and 12A. For example and as described for Figure 11A, all the valves 1302a 1302b 1302c are synchronized in motion. Even when one valve is faulty, there are other working valves that can prevent air from leaking from the valve control unit 1008.

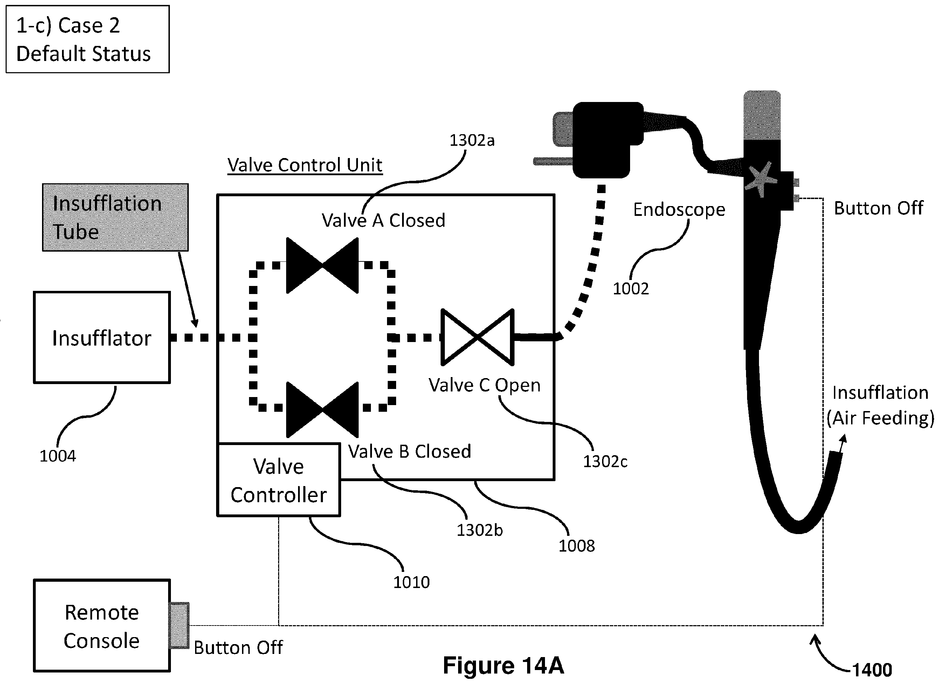

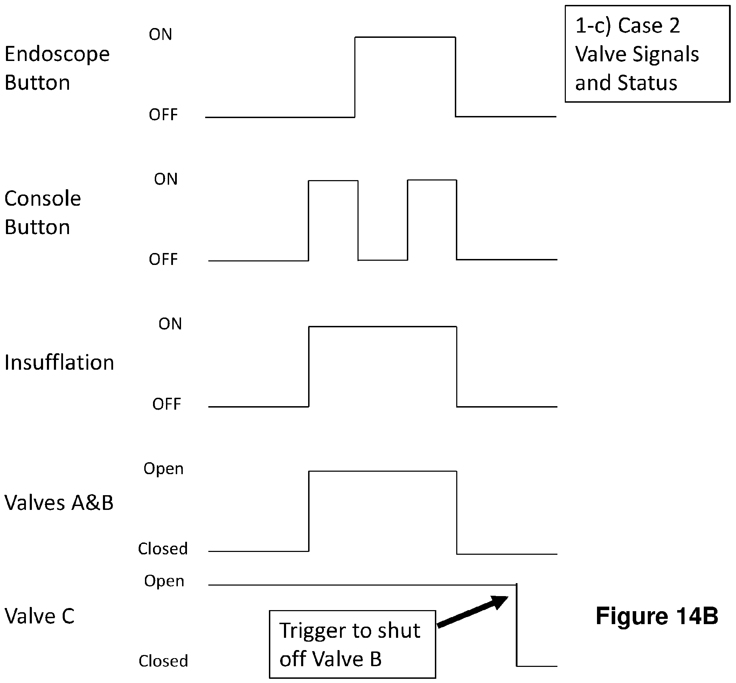

Figure 14A shows a schematic diagram 1400 of multiple valves in parallel and series in a valve control unit of an endoscopic system while Figure 14B shows a graphic representation of the valve signals and status of the valve control unit of Figure 14A according to an example embodiment. In the case as described in Figure 12A and as shown in Figure 14A, valve C 1302c remains open while valve A 1302a and valve B 1302b are closed. If either Valve A 1302a or valve B 1302b malfunctions, valve C 1302c is closed to prevent over-inflation.

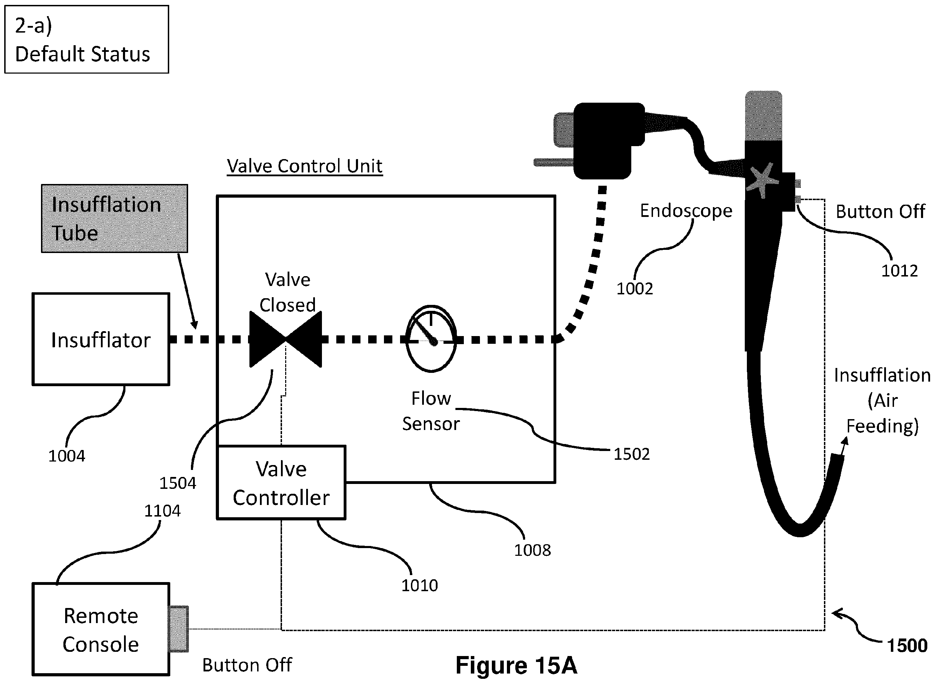

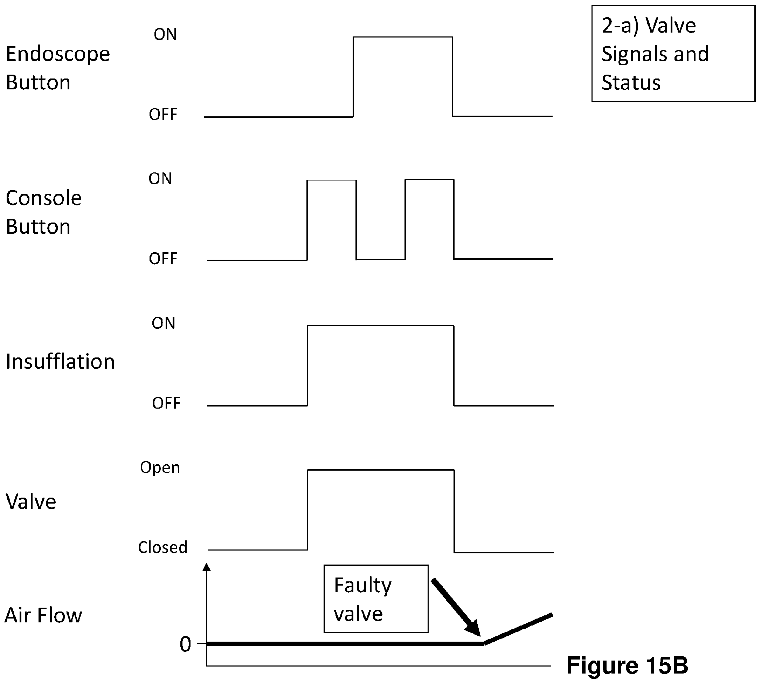

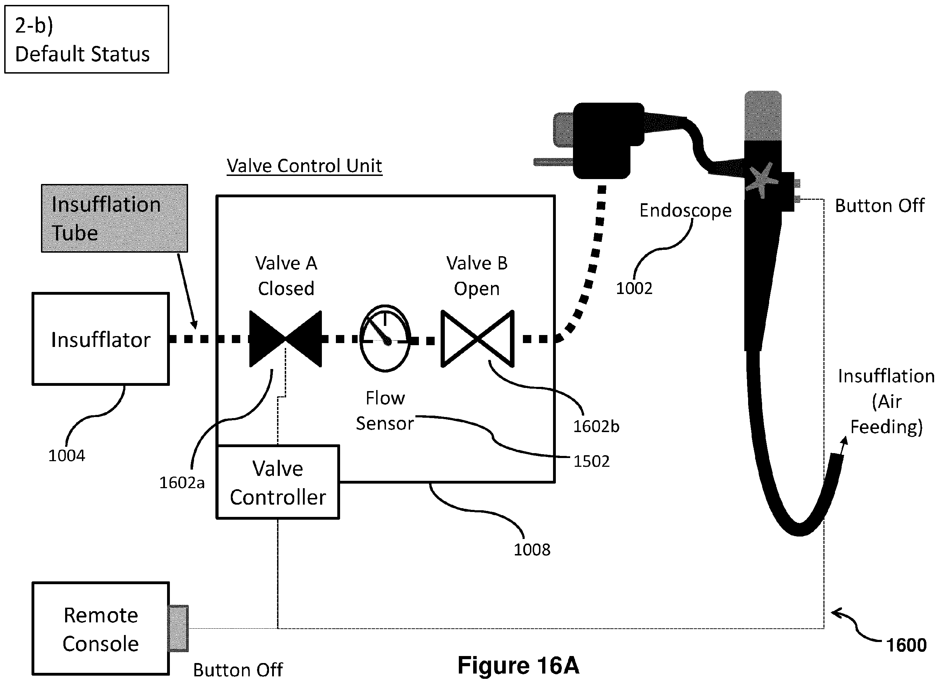

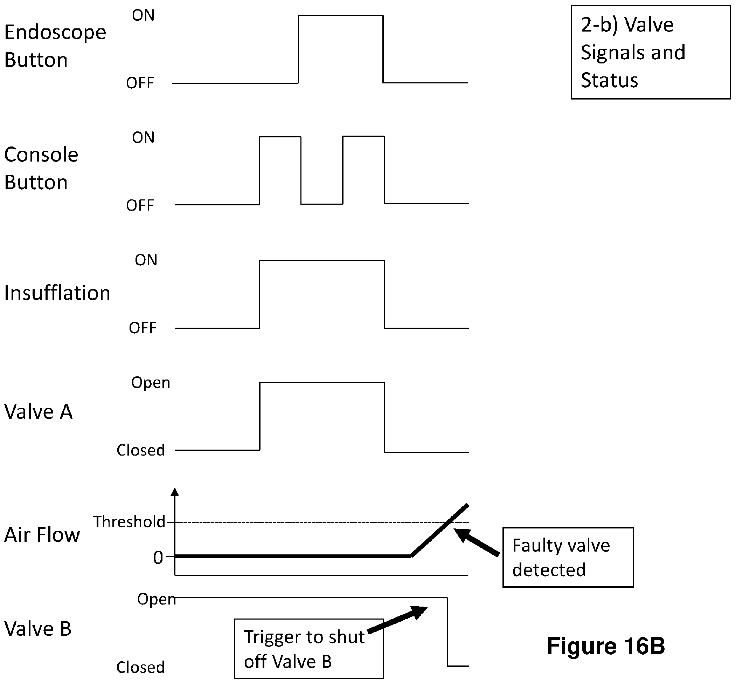

Figure 15A shows a schematic diagram 1500 of a valve control unit having a flow sensor in an endoscopic system while Figure 15B shows a graphic representation of the valve signals and status of the valve control unit of Figure 15A according to an example embodiment. As shown in the Figure, an air flow sensor 1502 is used to detect air leakage from a faulty valve. The air flow sensor 1502 can be placed in series behind a valve 1504 that controls air feeding from the insufflator 1004 into the patient's body. When the valve 1504 opens, air flow is detected by the flow sensor 1502. Therefore, if the valve 1504 is supposed to be closed and there is an indication of air flow by the sensor 1502, it may be an indication that the valve 1504 may be faulty and leaking air. The sensor 1502 may include a graphical and/or audio indicator on the Valve Control Unit 1008 to display the current air flow rate. It can be appreciated that the graphical and/or audio indicator may also be positioned at other places, such as the endoscope 1002 or the remote console 1104. The air flow sensor 1502 can be placed downstream relative to the valve 1504 and upstream relative to the endoscope 1002. Alternatively, the sensor 1502 may be also placed upstream relative to the valve 1504 and downstream relative to the insufflator 1004. An alternative embodiment of using the air flow sensor 1502 with two valves 1602a 1602b can be shown in Figure 16A and its corresponding graphic representation of the valve signals and status of the valve control unit is shown in Figure 16B.

With the addition of an accessory, a threshold for air flow may be predetermined. If the current measured air flow rate is less than the predetermined threshold, it may indicate a status of no air flow to the system. On the other hand, if the current measured air flow rate is higher than the predetermined threshold, it may indicate a status of positive air flow to the system. When the user (or endoscopist) is not pressing the air valve activation button 1012 either from the endoscope 1002 or the remote console 1104, but the system indicates air flow based on the flow sensor threshold, it may imply that there may be air leakage from the valve 1504. In such a case, a warning sign can be displayed to the user through a user interface or through an audio signal. Alternatively, a safety system may be implemented to shut off air flow automatically.

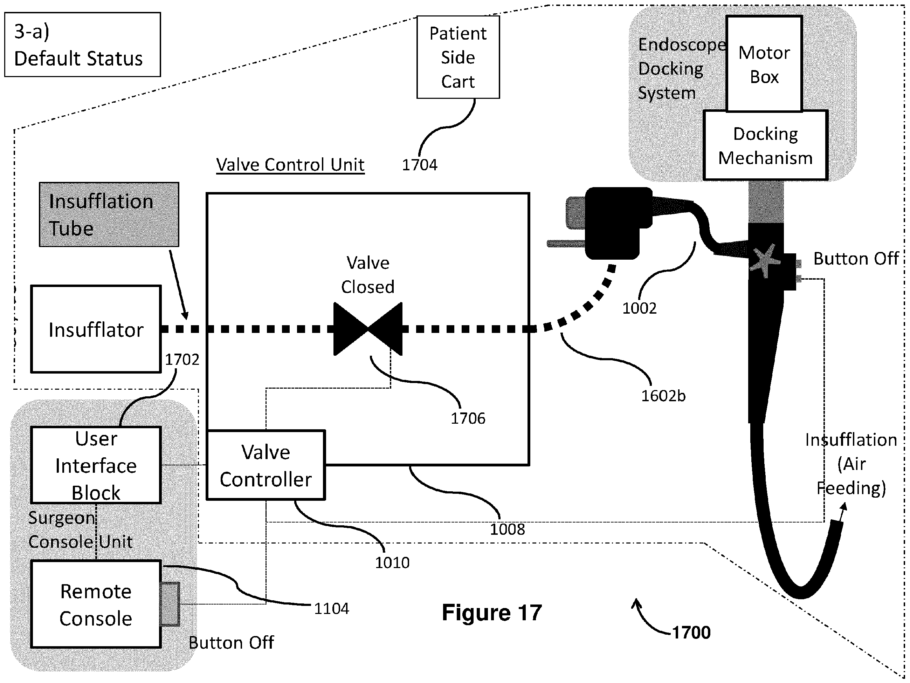

Embodiments of the present invention may also provide multi-user controllable valves to overcome improper insufflation control. An example of multi-user controllable valves include having user-controllable valve activation settings. Figure 17 shows a schematic diagram 1700 illustrating user-controllable valve activation settings in an endoscopic system according to an example embodiment. The endoscopic system may provide the capability to control the valve activation functions from both the endoscope 1002 and the remote console 1104, such that the user (or endoscopist) can be given an option to select what functions should be controlled from the endoscope 1002 and/or the remote console 1104. There are three options to do so, namely (i) only from the endoscope 1002, (ii) only from the remote console 1104, or (iii) from both the endoscope 1002 and the remote console 1104. In the third option, as long as one user sends a command to feed air, the valve controller 1010 sends air to the endoscope 1002. The settings can be controlled through a user interface 1702 on the remote console 1104 or a panel on the Valve Control Unit 1008 or the Patient Side Cart 1704. Alternatively, it can be pre-programmed by a manufacturer. One possible scenario is that the users can control the valve(s) 1706 only from the endoscope 1002 by default and are given an option to activate the valve control from the remote console 1104 so that they can be controlled from both sides. Another scenario is that the users have all the valve control from both the endoscope 1002 and the remote console 1104 by default and given a choice to deactivate certain valve control so it can be controlled only from one side, but not from the other. The remote control valve settings can be further broken down depending on the endoscope docking status. This means that the users can change valve control settings before and after docking the endoscope 1002 to the endoscopic system.

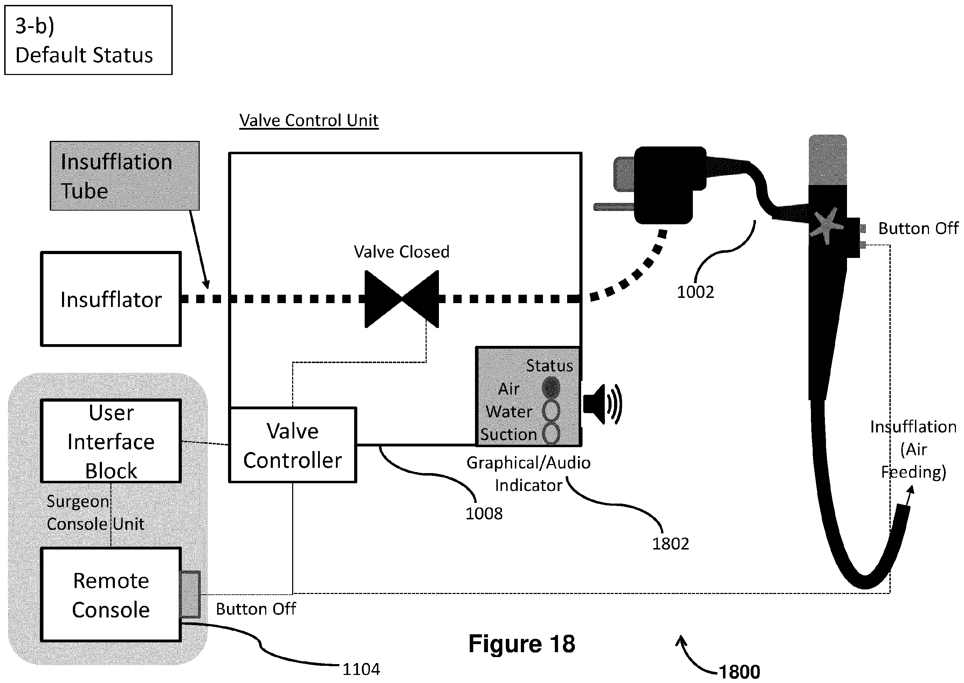

Further examples of multi-user controllable valves include having visual and/or audio indicators for valve activation. Figure 18 shows a schematic diagram 1800 illustrating visual and/or audio indicators in an endoscopic system according to an example embodiment. In an embodiment, a graphical and/or audio indicator 1802 may be included on the remote console 1104, Valve Control Unit 1008, and/or the Patient Side Cart to display if the valves are activated from the endoscope 1002 or the remote console 1104. For example and as shown in the Figure, the status of the air is indicated at the graphical and/or audio indicator 1802 by lighting up a bulb. The graphical and/or audio indicator 1802 may be positioned such that it is easily seen by the user (or endoscopist). In the Figure, the graphical and/or audio indicator 1802 is positioned at the valve control unit 1008. Similarly, an audible sound may be produced by the graphical and/or audio indicator 1802 to indicate a valve control button is pressed. The graphical and/or audio indicator 1802 may allow other users (endoscopists, nurses or assistants etc.) know when the valves are activated.

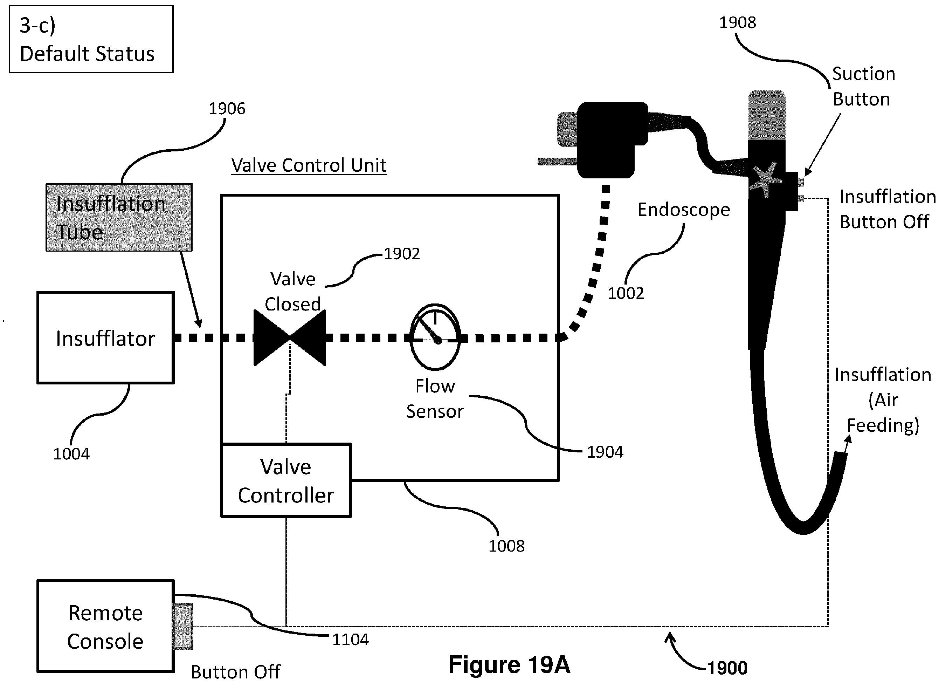

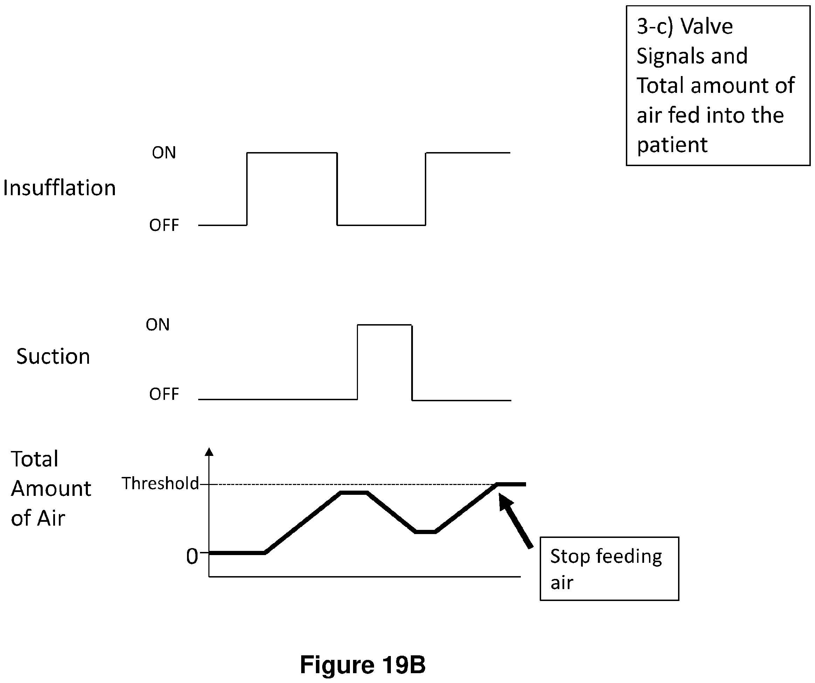

Yet another example of multi-user controllable valves include having an automatic shut-off function in the endoscopic system. Figure 19A shows a schematic diagram 1900 illustrating a suction button having in an endoscopic system while Figure 19B shows a graphic representation of the valve signals and the amount of air in the endoscopic system of Figure 19A according to an example embodiment. In an embodiment, if the air valve 1902 gets continuously activated by the user(s) for a long time, the system detects the continuous activation and automatically turns off the air feeding. For example, an air flow sensor 1904 can be used to determine if air is flowing through the insufflation tube 1906 for longer than a pre-determined time or above a pre-determined volume / level. The pre-determined value can be either continuous activation time (for example, more than 1 minute) or the total amount of air flow (for example, more than 3 cubic centimetres). If the predetermined value relates to the total amount of air flow, suction rate may be taken into consideration because suction lowers air pressure inside the gastrointestinal (GI) tract of the patient. In this case, a suction button 1908 may be provided at the endoscope 1002 to lower the air pressure such that the total air flow amount is decreased to the predetermined value. Further, a visual/audio alarm system can be used to warn the user rather than automatically shutting off the insufflation function. The air flow sensor 1904 can be placed downstream relative to the valve 1902 and upstream relative to the endoscope 1002 as shown in the Figure. Alternatively, the air flow sensor 1904 may be placed upstream relative to the valve 1902 and downstream relative to the insufflator 1004. The valve activation can be done from the endoscope 1002, or from the remote console 1104, or combination of the two.

An inlet seal for an endoscopic system may be required to provide air tightness while maintaining low friction and low manufacturing cost at the same time. In embodiments of the present invention, the inlet seal for surgical instrument channel may consist of structures as further discussed below. In an embodiment, the inlet seal may have a fixing structure to the surgical instrument channel inlet on the endoscope. The inlet seal may also have a seal to keep airtightness between the inlet seal and the surgical instrument inlet. The inlet seal may include two or more seals. The first seal works during endoscope insertion without the surgical instruments while the second seal works during Endoscope Submucosal Dissection (ESD) operation and endoscope extraction with the surgical instruments inserted. The first seal may consist of a plastic material such as paper, film, resin, rubber and gel, and can be disposable such that the surgical instrument may break the first seal when the surgical instrument is inserted. In addition, each of the two seals may be able to impregnate a lubricant such as silicone oil. The first seal may be symmetrically-shaped to make friction though instrument insertion and extraction at the same level. The inlet seal may also be mounted on the surgical instrument inlet with a snap fit or screw. In a surgical instrument having a plurality of surgical instrument channel (including its inlet), the two seals of the inlet seal can be combined with each other.

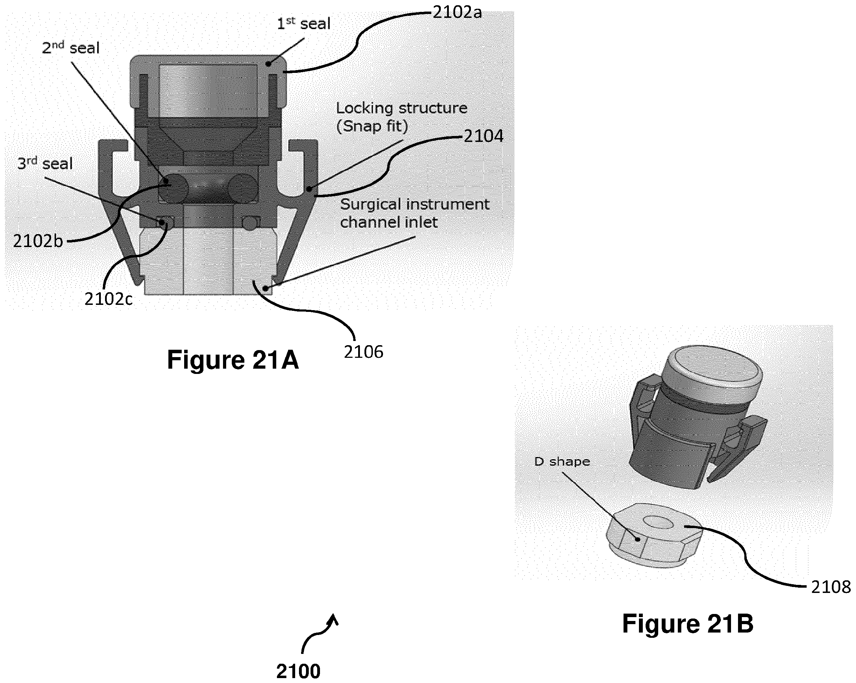

Figure 21A shows a cross section of an inlet seal locking structure in an endoscopic system while Figure 21B shows a perspective view of the inlet seal locking structure of Figure 21A according to an example embodiment. As shown in Figure 21A, the inlet seal may include three seals 2102a 2102b 2102c to keep airtightness between the inlet seal and a surgical instrument channel inlet 2106. The locking structure 2104 may be coupled to the channel inlet 2106 with fixed force which is higher than the maximum friction force between a surgical instrument and the seal while a surgical instrument is translating. In an embodiment, the inlet seal can be mated onto a fixing structure such as a groove formed on the surgical instrument channel inlet 2106 with a snap fit on its body. In such a structure, the mating structure may consist of a D shape 2108 and grooves to prevent shifting of the inlet seal.

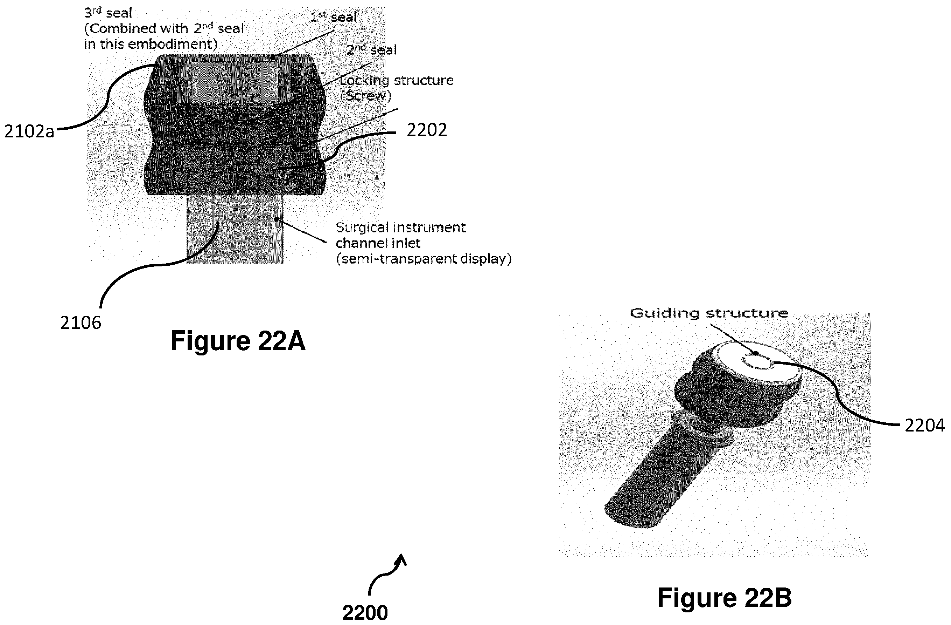

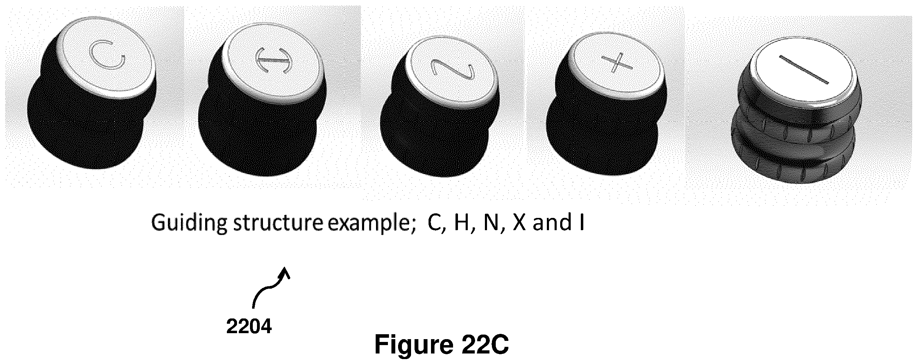

Figure 22A shows a cross section of an inlet seal locking structure having a screw in an endoscopic system while Figure 22B shows a perspective view of the inlet seal locking structure of Figure 22A according to an example embodiment. In the Figures, the inlet seal may include a screw 2202 on the bottom of its body to mate with the surgical instrument channel inlet 2106. The first seal 2102a of the inlet seal may keep airtightness without having a surgical instrument. The first seal 2102a may be designed for airtightness though the endoscope insertion and is broken when a surgical instrument is inserted. The first seal 2102a can include a guiding structure 2204 which makes it to be easily broken and as when desired. This is so as not to disturb an insertion and extraction of the surgical instruments. The shape of the guiding structure 2204 may preferably not include a separated shape so as to prevent seal debris from dropping into human body during an endoscopy procedure. For example and as shown in Figure 22C, the guiding structure 2204 may preferably be an open line shape such as; C, H, N, X and I.

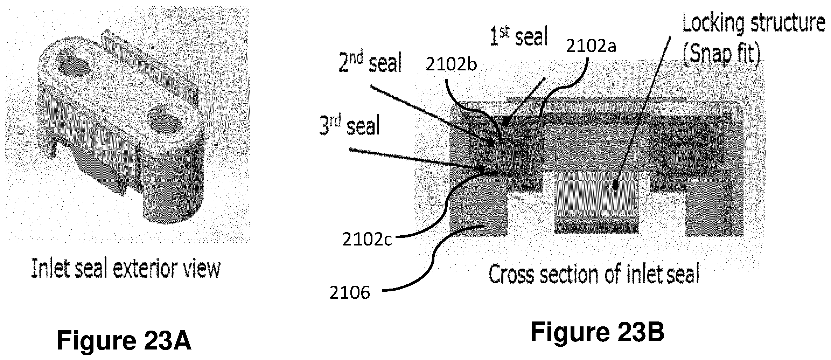

Figure 23A shows a perspective view of an exterior of the inlet seal in an endoscopic system while Figure 23B shows a cross section of the inlet seal of Figure 23A according to an example embodiment. In an embodiment, the second seal 2102b of the inlet seal may be provided to keep airtightness with a surgical instrument such that the second seal 2102b airtightness when a surgical instrument is inserted. The third seal 2102c may be positioned between the channel inlet 2106 and the inlet seal such that the third seal 2102c may provide airtightness between the surgical instrument channel inlet 2106 and the inlet seal itself. In an embodiment, the second seal 2102b and the third seal 2102c can be formed into one body.

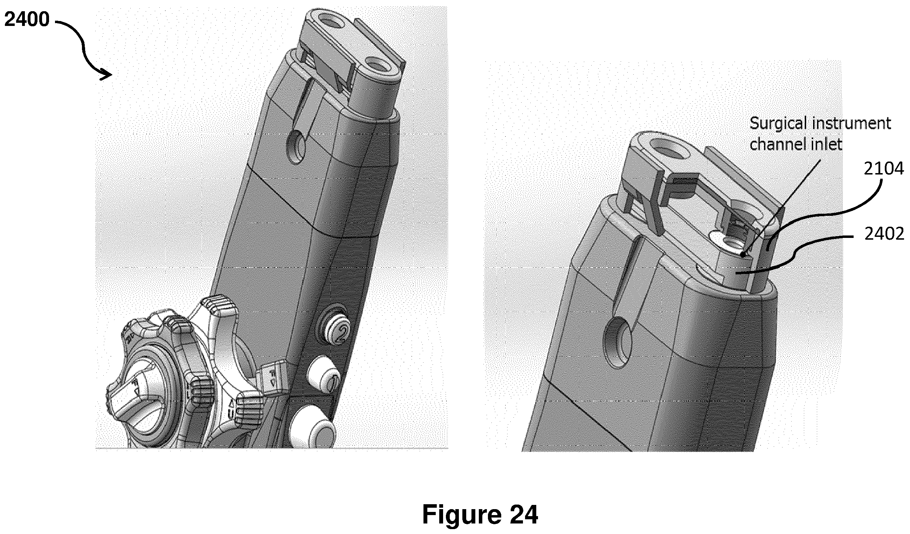

Figure 24 shows perspective views of an inlet seal on an endoscope having a plurality of surgical instrument channels according to an example embodiment. In the Figure, an endoscope may include a plurality of surgical instrument channels 2402. In this embodiment, the inlet seal may include a plurality of the first, second and third seals (not shown) together with the locking structure 2104. At one time to set up the inlet seal, all of the plurality of surgical instrument channels can be made with airtight sealing. Advantageously, the first seal which is formed by not sticky material like as a paper, may have less friction with the surgical instrument because of the low elasticity. Further, the overall structure may be simple and the inlet seal can be assembled inexpensively.

According to an aspect of the invention, there is provided an endoscope system comprising an endoscope having a hollow tube formed therein; and an instrument for insertion through the hollow tube, wherein the instrument has a first end for operational control and a second distal end for instrument operation at a distal end of the endoscope, wherein the distal end of the endoscope has an illumination device and a vision device for providing illuminated vision at the distal end of the endoscope for operational control of the instrument operation at the distal end of the instrument, and wherein the distal end of the instrument and the distal end of the endoscope are interoperably coupled for rotational control of the instrument with respect to the endoscope.

In an embodiment, the system may include a divot formed at a predetermined distance from the distal end of a first one of the instrument or the endoscope, and wherein a nipple is located the predetermined distance from the distal end of a second one of the instrument or the endoscope such that the divot and the nipple interoperably couple together when the instrument and the endoscope are oriented in a predetermined manner for mechanical prevention of further rotation of the instrument with respect to the endoscope, and wherein one or both of the divot and the nipple are compliantly flexible.

In an embodiment, the system may include a nipple located at the predetermined distance from the distal end of the endoscope and a flexible portion of the inner surface of the endoscope is located a predetermined distance from the distal end of the endoscope, the system further comprising a divot located the predetermined distance from the distal end of the instrument such that the nipple and the divot interoperably couple together with the flexible portion of the inner surface disposed therebetween when the instrument and the endoscope are oriented in a predetermined manner for mechanical prevention of further rotation of the instrument with respect to the endoscope, and wherein the compliant surface comprises an integral fluid-tight flexible portion of the inner surface of the hollow tube of the endoscope.

In an embodiment, the endoscope may comprise an optical sensor system located a predetermined distance from the distal end of the endoscope, the optical sensor system comprising an optical emitter and an optical receiver, and wherein an optical interactive device is located on or in the instrument the predetermined distance from the distal end of the instrument such that the optical sensor system and the optical interactive device operate together to generate an orientation signal indicating an orientation of the instrument with respect to the endoscope.

In an embodiment, the optical emitter may be located within the endoscope to emit light of a predetermined frequency or frequencies from a first internal side and wherein the optical receiver is located within the endoscope to receive light of the predetermined frequency or frequencies at a second side of the endoscope opposite the first side, and wherein the optical interactive device comprises a more or less light transmissive portion of the instrument as compared to other portions of the instrument, and wherein the optical sensor system operates with the optical interactive device to generate an orientation signal indicating an orientation of the instrument with respect to the endoscope in response to the light from the optical emitter passing through the more or less light transmissive portion of the instrument to the optical receiver.

In an embodiment, the optical emitter may be located within the endoscope to emit light of a predetermined frequency or frequencies from a first internal side and wherein the optical receiver is located within the endoscope to receive light of the predetermined frequency or frequencies at a second side of the endoscope opposite the first side, and wherein the optical interactive device comprises an optically interactive portion of the instrument that diffuses, redirects or imparts only a predetermined polarization of light as compared to other portions of the instrument, and wherein the optical sensor system operates with the optical interactive device to generate an orientation signal indicating an orientation of the instrument with respect to the endoscope in response to the diffused, redirected or repolarized light from the optical emitter passing through the optically interactive portion of the instrument to the optical receiver.

In an embodiment, the optical emitter and the optical receiver are located within the endoscope to emit light of a predetermined frequency or frequencies from a first internal side and receive light of the predetermined frequency or frequencies at the first internal side of the endoscope, and wherein the optical interactive device comprises a more or less light reflective device on or portion of the instrument as compared to other portions of the instrument, and wherein the optical sensor system operates with the optical interactive device to generate an orientation signal indicating an orientation of the instrument with respect to the endoscope in response to the light from the optical emitter reflecting off the more or less light reflective device on or portion of the instrument to the optical receiver.

In an embodiment, the optical emitter and the optical receiver are located within the endoscope to emit light of a predetermined frequency or frequencies from a first internal side and receive light of the predetermined frequency or frequencies at the first internal side of the endoscope, and wherein the optical interactive device comprises an optically interactive portion of the instrument that diffuses, redirects or imparts only a predetermined polarization of light as compared to other portions of the instrument, and wherein the optical sensor system operates with the optical interactive device to generate an orientation signal indicating an orientation of the instrument with respect to the endoscope in response to the diffused, redirected or repolarized light from the optical emitter reflecting from the optically interactive portion of the instrument to the optical receiver.

In an embodiment, the optical emitter and the optical receiver are located within the endoscope behind an integral fluid-tight light transmissive portion of the inner surface of the hollow tube of the endoscope.

In an embodiment, the endoscope further comprises a magnetic field sensing system located at the predetermined distance from the distal end of the endoscope and the instrument, and wherein the magnetic field sensing system includes a magnetic field sensor and a magnetic field producer operably coupled to generate an orientation signal indicating an orientation of the instrument with respect to the endoscope.

In an embodiment, the magnetic field sensor comprises one or more of a hall-effect sensor and a magnetometer.

In an embodiment, the magnetic field producer comprises one or more of a permanent magnet and a coil of wire that is operably energizable to produce a magnetic field.

In an embodiment, the endoscope comprises the magnetic field sensor, and wherein the magnetic field producer is aligned on or within the instrument, and wherein a change in orientation of the instrument with respect to the endoscope will result in a detectible change of magnetic field strength or magnetic field direction at the magnetic field sensor in the endoscope.

In an embodiment, the instrument comprises the magnetic field sensor, and wherein the magnetic field producer is aligned on or within the endoscope, and wherein a change in orientation of the instrument with respect to the endoscope will result in a detectible change of magnetic field strength or magnetic field direction at the magnetic field sensor in the instrument.

In an embodiment, the endoscope comprises a detachable hood at the distal end of the endoscope, and wherein the distal end of the instrument and the detachable hood are interoperably coupled for rotational control of the instrument with respect to the endoscope.

In an embodiment, a ditch is formed in the instrument a predetermined distance from the distal end of the instrument and wherein a spring pin is located in the detachable hood the predetermined distance from the distal end of the detachable hood such that the spring pin and the ditch interoperably engage when the instrument and the endoscope are oriented in a predetermined manner for mechanical prevention of further rotation of the instrument with respect to the endoscope.

In an embodiment, the system further comprises two or more spring pins arranged in a longitudinal direction of a channel provided on the detachable hood, and including a plurality of guide components forming a ditch on the instrument, wherein the plurality of guide components comprise a plurality of ring-shaped rigid components and located within a predetermined distance of one another, wherein in the case where a length of a ditch made on each of the plurality of ring-shaped rigid component is L1, a distance between two adjacent ring-shaped rigid components is L2 and a distance between two adjacent spring pins is L3, then L1 is larger than L3 and that L3 is larger than L2 (L1 > L3 > L2).

In an embodiment, a leaf spring is located on the instrument a predetermined distance from the distal end of the instrument and wherein a ditch is formed in the detachable hood the predetermined distance from the distal end of the detachable hood such that the leaf spring and the ditch interoperably engage when the instrument and the endoscope are oriented in a predetermined manner for mechanical prevention of further rotation of the instrument with respect to the endoscope.

In an embodiment, a ditch is formed in the instrument a predetermined distance from the distal end of the instrument and wherein a compliant rolling element is located on the detachable hood the predetermined distance from the distal end of the detachable hood such that the compliant rolling element and the ditch interoperably engage when the instrument and the endoscope are oriented in a predetermined manner for mechanical prevention of further rotation of the instrument with respect to the endoscope.

In an embodiment, the compliant rolling element comprises a rolling element, and wherein a compliability of the compliant rolling element is provided by one or both of a resilient material disposed symmetrically around an outer surface of the rolling element and a compliant axle of the rolling element.

In an embodiment, the rolling element comprises a bearing or a bushing.

In an embodiment, the compliant axle of the rolling element comprises spring loaded elements to provide the compliability to the rolling element.

In an embodiment, a bump is formed on the instrument a predetermined distance from the distal end of the instrument and wherein a C-spring is located in the detachable hood the predetermined distance from the distal end of the detachable hood such that the bump and the C-spring interoperably engage when the instrument and the endoscope are oriented in a predetermined manner for mechanical prevention of further rotation of the instrument with respect to the endoscope.

In an embodiment, a rotation hampering device is located in the detachable hood, the rotation hampering device imparting high friction against the instrument when the instrument attempts to move rotationally within the rotation hampering device while imparting less friction against the instrument when the instrument attempts to move translationally within the rotation hampering device for reduction of rotation of the instrument with respect to the endoscope.

In an embodiment, the rotation hampering device comprises an elastomeric tube with teeth disposed around an internal circumference of the elastomeric tube, wherein the teeth make contact with the instrument and impart a substantially normal force onto a surface of the instrument, and wherein the teeth of the elastomeric tube are stiffer when bending in a rotation direction than when bending in a translation direction.

In an embodiment, the rotation hampering device comprises a left hand coil located in the detachable hood and a right hand coil also located in the detachable hood, the left hand coil and the right hand coil functioning together as an anti-rotation clutch such that the left hand coil imparts high friction against the instrument when the instrument attempts to rotate to the right and the right hand coil imparts high friction against the instrument when the instrument attempts to rotate to the left, the left hand coil and the right hand coil imparting less friction against the instrument when the instrument attempts to move translationally.

In an embodiment, an engageable device is formed on the instrument a predetermined distance from the distal end of the instrument and wherein a corresponding structure is located on the detachable hood the predetermined distance from the distal end of the detachable hood such that the engageable device and the corresponding structure interoperably engage when the instrument is extended through the endoscope a certain distance and oriented in a predetermined manner for mechanical prevention of further rotation of the instrument with respect to the endoscope.

In an embodiment, a length of the corresponding structure is longer than a distance between two adjacent engageable devices.

It will be appreciated by a person skilled in the art that numerous variations and/or modifications may be made to the present invention as shown in the embodiments without departing from a spirit or scope of the invention as broadly described. The embodiments are, therefore, to be considered in all respects to be illustrative and not restrictive.

## Claims

1. An insufflation control device for an endoscope system, comprising:
a master valve that is disposed downstream relative to an insufflator of the endoscope system and upstream relative to an endoscope of the endoscope system;
a slave valve that is disposed downstream relative to the insufflator and upstream relative to the endoscope; and
a valve controller that is configured to operationally control one or more of the master valve or the slave valve in response to commands received either from the endoscope system or from a remote console,
wherein the master valve and the slave valve are disposed in series with each other.

2. The device in accordance with claim 1, wherein the valve controller is configured to operationally control both the master valve and the slave valve in response to the commands received either from the endoscope system or from the remote console, such that under normal operating conditions both the master valve and the slave valve are open or closed at substantially the same time.

3. The device in accordance with claim 1 or 2, wherein the valve controller is configured to operationally control only the master valve in response to the commands received either from the endoscope system or from the remote console, wherein under the normal operating conditions the slave valve is open and wherein the valve controller is configured to close the slave valve in response to an override command.

4. The device in accordance with claim 1, wherein the device further comprises an additional master valve that is disposed downstream relative to the insufflator, upstream relative to the endoscope, and in parallel with the master valve,
and wherein the valve controller is further configured to operationally control the master valve, the slave valve and the additional master valve in response to the commands received either from the endoscope system or from the remote console, such that under the normal operating conditions the master valve, the slave valve and the additional master valve are open or closed at substantially the same time.

5. The device in accordance with claim 4, wherein the valve controller is configured to operationally control only the master valve and the additional master valve in response to the commands received either from the endoscope system or from the remote console, wherein under the normal operating conditions the slave valve is open and wherein the valve controller is configured to close the slave valve in response to an override command.

6. The device in accordance with claim 5, wherein the device further comprises an air flow sensor disposed in series with the master valve, downstream relative to the master valve and upstream relative to the endoscope, wherein the air flow sensor and the master valve are in fluid communication with an insufflation tube of the endoscope system, and wherein the air flow sensor is configured to provide an indication of air flow through the insufflation tube.

7. The device in accordance with claim 6, wherein the device further comprises a valve control unit, the valve control unit with the air flow sensor is further configured to provide an alert on a condition that the air flow through the insufflation tube that is determined by the air flow sensor is above a pre-determined level, which implies air leakage from the valve.

8. An insufflation control device for an endoscope system, comprising:
a valve that is disposed downstream relative to an insufflator of the endoscope system and upstream relative to an endoscope of the endoscope system;
a valve controller that is configured to operationally control the valve in response to commands received from the endoscope system, and/or configured to operationally control the valve in response to commands received from a remote console on a condition that a switch command is received at the valve controller.

9. The device in accordance with claim 8, wherein the switch command is transmitted from an input module associated with the endoscope system to the valve controller in response to a user instruction to switch control from the endoscope system to the remote console and vice versa.

10. The device in accordance with claim 9, wherein the switch command is transmitted from an input module associated with the remote console to the valve controller in response to a user instruction to switch control from the endoscope system to the remote console and vice versa.

11. An insufflation control device for an endoscope system, comprising:
a valve that is disposed downstream relative to an insufflator of the endoscope system and upstream relative to an endoscope of the endoscope system;
a valve controller that is configured to operationally control the valve either in response to commands received from the endoscope system or from a remote console on a condition that an interlock command is received at the valve controller.

12. The device in accordance with claim 11, wherein the interlock command is transmitted from a docking module of the endoscope system to the valve controller based on a docking status of the endoscope.

13. The device in accordance with claim 11, wherein the device further comprises an output module that is in communication with (i) the valve, (ii) the input module associated with the endoscope system, and/or (iii) the input module associated with the remote console, wherein the output module is configured to provide an indication of:
an operational status of the valve;
a presence of the command received from the endoscope system; and/or a presence of the command received from the remote console.

14. The device in accordance with claim 13, wherein the device further comprises an air flow sensor disposed in series with the valve, downstream relative to the valve and upstream relative to the endoscope, wherein the air flow sensor and the master valve are in fluid communication with an insufflation tube of the endoscope system, and wherein the air flow sensor is configured to transmit a close command to the valve controller on a condition that the air flow through the insufflation tube that is determined by the air flow sensor is above a pre-determined level or is present for longer than a pre-determined time.
